(19) Europäisches Patentamt
European Patent Office
Office européen des brevets

(11) **EP 3 568 074 B1**

(12) **FASCICULE DE BREVET EUROPEEN**

(45) Date de publication et mention de la délivrance du brevet:
**02.07.2025 Bulletin 2025/27**

(21) Numéro de dépôt: **18700200.1**

(22) Date de dépôt: **09.01.2018**

(51) Classification Internationale des Brevets (IPC):
***A61B 5/00*** *(2006.01)* ***A61B 5/11*** *(2006.01)*
***G01C 22/00*** *(2006.01)*

(52) Classification Coopérative des Brevets (CPC):
**G01C 22/006; A61B 5/112; A61B 5/4082; A61B 5/6829; A61B 5/7246;** A61B 2562/0219

(86) Numéro de dépôt international:
**PCT/EP2018/050450**

(87) Numéro de publication internationale:
**WO 2018/130521 (19.07.2018 Gazette 2018/29)**

(54) **LOGICIEL LOCOGRAMME: OUTIL POUR ANALYSER LES EXERCICES DE MARCHE**

LOKOGRAMM-SOFTWARE WERKZEUG ZUR ANALYSE VON GEHÜBUNGEN

LOCOGRAM SOFTWARE: TOOL FOR ANALYSING WALKING EXERCISES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.01.2017 FR 1770031**

(43) Date de publication de la demande:
**20.11.2019 Bulletin 2019/47**

(73) Titulaires:
- **Université Paris Cité**
  **75006 Paris (FR)**
- **Centre National de la Recherche Scientifique**
  **75794 Paris Cedex 16 (FR)**
- **UNIVERSITE PARIS NORD**
  **93430 Villetaneuse (FR)**
- **Etat Français - Ministère de la Défense - Direction centrale du service de santé des armées**
  **75614 Paris Cedex 12 (FR)**

(72) Inventeurs:
- **VIDAL, Pierre**
  **75005 Paris (FR)**
- **BARROIS-MULLER, Rémi**
  **75018 Paris (FR)**
- **RICARD, Damien**
  **92140 Clamart (FR)**
- **OUDRE, Laurent**
  **75012 Paris (FR)**

(74) Mandataire: **A.P.I. Conseil**
**Immeuble Newton**
**4, rue Jules Ferry**
**64000 Pau (FR)**

(56) Documents cités:
- **YANG MINGJING ET AL: "iGAIT: An interactive accelerometer based gait analysis system", COMPUTER METHODS AND PROGRAMS IN BIOMEDICINE, vol. 108, no. 2, 2012, pages 715 - 723, XP028974078, ISSN: 0169-2607, DOI: 10.1016/J.CMPB.2012.04.004**
- **MOE-NILSSEN R ET AL: "Estimation of gait cycle characteristics by trunk accelerometry", JOURNAL OF BIOMECHANICS, PERGAMON PRESS, NEW YORK, NY, US, vol. 37, no. 1, 2004, pages 121 - 126, XP004895603, ISSN: 0021-9290, DOI: 10.1016/S0021-9290(03)00233-1**
- **CHE-CHANG YANG ET AL: "Real-Time Gait Cycle Parameter Recognition Using a Wearable Accelerometry System", SENSORS, vol. 11, no. 12, 25 July 2011 (2011-07-25), pages 7314 - 7326, XP055364449, DOI: 10.3390/s110807314**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Processed by Luminess, 75001 PARIS (FR)

## Description

La présente invention concerne un procédé et un dispositif permettant l'analyse de la régularité et de la symétrie d'une séquence de cycles de marche ou de course d'une personne.

## ETAT DE LA TECHNIQUE

Récemment, le progrès de l'électronique et de l'informatique a entraîné le développement de nouveaux capteurs qui rendent la mesure de la marche en consultation clinique de routine possible (par exemple à partir de capteurs inertiels). Ces capteurs donnent accès à des signaux qu'il faut synthétiser pour en tirer des conclusions sur la marche d'un sujet.

Les paramètres de marche comme la vitesse ou la cadence constituent la forme la plus synthétisée des signaux de marche car ils les réduisent à un nombre. Ainsi, les constructeurs d'outils de mesure du pas fournissent des compte-rendus exhaustifs sous forme de liste de paramètres traduisant les différents aspects de la marche d'un sujet comme la symétrie, la régularité ou la rapidité de la mise en route.

Il peut y avoir plus d'une centaine de paramètres. Ils sont représentés de manière adaptée sous forme de tableau, de diagramme en en barre ou de courbe.

Néanmoins, la thématique de la visualisation des données intermédiaires entre les signaux temporels bruts ininterprétables et les paramètres est peu explorée.

Certains paramètres nécessitent la définition de seuils pour être calculés. Ainsi, on définit par exemple qu'un sujet a atteint un régime établi lorsque l'amplitude de ses cycles a dépassé 67% de l'amplitude des cycles n°5 à 10 (on considère qu'il n'y a plus lieu de parler d'initiation à plus de 5 cycles). Certains cycles peuvent avoir des valeurs limites (60% ou 70% de l'amplitude des cycles à distance).

On commet ainsi des erreurs liées au seuil choisi qui pourraient être évitées en regardant directement la donnée brute.

Les paramètres moyennent les caractéristiques de la marche sur l'ensemble de l'exercice, et occultent de ce fait le déroulé de l'exercice. Par exemple, pour évaluer la régularité de la marche d'un sujet, on utilise la déviation standard de la durée des cycles de marche. Si les cycles sont globalement réguliers et qu'un cycle se trouve plus long que les autres, la déviation standard sera augmentée. On ne saura pas que cette augmentation sera due à un seul cycle isolé, ni à quel moment de la marche celui-ci a eu lieu. Or, le nombre de cycles erratiques, et leur instant de survenu sont des informations analytiques importantes.

Les paramètres sont des bons indicateurs de la performance de la marche d'un sujet. Ils n'apportent néanmoins pas d'information analytique sur l'origine de la modification de la marche. Par exemple, la vitesse de marche est une bonne évaluation globale, cependant, une vitesse peut être diminuée par des pas plus courts ou une cadence diminuée. A leur tour, la taille des pas et la cadence peuvent être affectés par différents facteurs.

La mesure de la marche présente d'importantes fluctuations interindividuelles dues principalement aux variabilités anatomiques et fonctionnelles de chaque individu qui influent sur le positionnement des capteurs, et aux variabilités de style locomoteur de chaque individu. Les paramètres sont affectés par cette variabilité et il n'existe pas de méthode de référence pour s'en affranchir.

Le document MOE-NILSSEN R ET AL, "Estimation of gait cycle characteristics by trunk accelerometry", JOURNAL OF BIOMECHANICS, PERGAMON PRESS, NEW YORK, NY, US, (2004), vol. 37, no. 1, doi:10.1016/S0021-9290(03)00233-1, ISSN 0021-9290, pages 121 -126, XP004895603, est cité pour définir l'état général de la technique.

Le document CHE-CHANG YANG ET AL, "Real-Time Gait Cycle Parameter Recognition Using a Wearable Accelerometry System", SENSORS, (20110725), vol. 11, no. 12, doi:10.3390/s110807314, pages 7314 - 7326, XP055364449, est cité pour définir l'état général de la technique.

Le document YANG MINGJING ET AL, "iGAIT: An interactive accelerometer based gait analysis system", COMPUTER METHODS AND PROGRAMS IN BIOMEDICINE, (2012), vol. 108, no. 2, doi:10.1016/J.CMPB.2012.04.004, ISSN 0169-2607, pages 715 - 723, XP028974078, divulgue un dispositif et un procédé permettant par une représentation visuelle l'analyse de la régularité et de la symétrie d'une séquence de N cycles de marche ou de course d'une même personne, le dispositif comportant des capteurs de mesure de signaux temporels bruts inertiels ou relatifs à une grandeur physique d'un segment anatomigue de la personne, et une unité de traitement et de calcul, reliée aux capteurs de mesure.

## EXPOSE DE L'INVENTION

Il existe un besoin dans l'état de l'art de disposer d'un procédé qui permet d'étudier temporellement la régularité et la symétrie d'une séquence de cycles de marche ou de course d'une personne.

Un but de l'invention est de permettre de prendre en compte la forme du signal pour rendre compte de la régularité de la marche ou de course, tout en s'affranchissant de la variabilité de la durée ou de l'amplitude des cycles de marche ou de la course.

Dans l'invention, il est construit une matrice ou tableau de visualisation d'un exercice de marche ou de course. Cette matrice de visualisation M(i,j) est dite « locogramme », et est une matrice de Gram au sens ou xij=xji avec xij étant la valeur située à la ième ligne et à la jème colonne dans la matrice M(i,j).

Le locogramme est robuste au type de capteurs utilisés (accéléromètres, gyroscope, traceur infra-rouge, ainsi qu'à leur localisations anatomiques pour l'enregistrement de la marche (pied, jambe, ceinture, poignet, tête).

Grâce à la matrice de visualisation de l'invention, le professionnel qui analyse la marche (médecin, kiné, infirmière, podologue, coach, entraîneur sportif, concepteur de chaussures etc...) peut avoir une vue globale de l'exercice de marche à partir de laquelle il peut faire la sémiologie afin de se faire une idée de la qualité de la marche d'un patient et repérer aisément dans un exercice donné des pas atypiques. Les seuls paramètres ne suffisent pas pour avoir du patient un aperçu satisfaisant. La matrice de visualisation est à mi-chemin entre les paramètres et les signaux temporels bruts, et donne accès au médecin aux données de la marche du patient.

La matrice de visualisation est fondée sur la division de la marche ou de course en cycle. Il s'agit de comparer les cycles entre eux à l'aide de « distances » ou métriques mathématiques. En fonction du choix de la distance, tel ou tel aspect du signal va être comparé.

La présente invention concerne un procédé tel que défini dans les revendications ci-jointes.

La présente invention concerne aussi un dispositif tel que défini dans les revendications ci-jointes.

## DESCRIPTION DES FIGURES

D'autres objectifs, caractéristiques et avantages sortiront de la description détaillée qui suit en référence aux dessins donnés à titre illustratif et non limitatif parmi lesquels :

- la figure 1a représente le dispositif selon l'invention ;
- la figure 1b représente le procédé selon l'invention ;
- la figure 2 représente la définition des termes amplitude, durée et forme des cycles de marche ou de course ;
- la figure 3 représente l'outil de visualisation selon l'invention du cycle de marche d'un pied d'une personne sur un exercice de marche donné comportant un aller et retour sur une distance définie;
- les figures 4a et 4b représentent l'outil de visualisation F(i,j) qui se rapporte au coefficient de similarité de forme selon l'invention pour un sujet sain, respectivement pour un sujet parkinsonien, comparant pour chaque sujet, la forme de ses cycles de marche sur un exercice de marche donné comportant un aller et retour sur une distance définie;
- les figures 5a et 5b représentent l'outil de visualisation de la marche ou matrice carrée A(i,j) qui se rapporte au coefficient de similarité de l'amplitude selon l'invention pour un sujet sain, respectivement pour un sujet parkinsonien, comparant pour chaque sujet, l'amplitude de ses cycles de marche sur un exercice de marche donné comportant un aller et retour sur une distance définie;
- les figures 6a et 6b représentent l'outil de visualisation de la marche ou matrice carrée D(i,j) qui se rapporte au coefficient de similarité de durée selon l'invention pour un sujet sain, respectivement pour un sujet parkinsonien, comparant pour chaque sujet, la durée de ses cycles de marche sur un exercice de marche donné comportant un aller et retour sur une distance définie;
- la figure 7 représente, en fonction du temps, les signaux temporels bruts de l'accélération issus d'un accéléromètre triaxial selon les trois axes x, y et z, pour le pied droit d'une personne en mouvement sur un exercice de marche donné comportant un aller et retour sur une distance définie;
- la figure 8 représente, en fonction du temps, les signaux temporels bruts de l'accélération sans gravité correspondant aux signaux bruts de la figure 1 auxquels on a soustrait les signaux temporels bruts issus de l'accéléromètre triaxial selon les trois axes x, y et z pour le pied droit d'une personne immobile ;
- la figure 9 représente, en fonction du temps, la norme de l'accélération sans gravité du pied droit de la personne sur un exercice de marche donné comportant un aller et retour sur une distance définie;
- les figures 10a, 10b représentent, en fonction du temps, la norme de l'accélération sans gravité du pied droit de la personne, la norme de l'accélération sans gravité du pied gauche de la personne et le repérage des temps $\tau$_droit et $\tau$_gauche correspondant aux cycles de marche C1, C2....Cnd+ng sur un exercice de marche donné comportant un aller et retour sur une distance définie;
- la figure 10c représente le ré-échantillonnage des cycles de marche C1, C2....Cnd+ng.

Les figures présentées dans ce document ont uniquement une valeur illustrative.

## DESCRIPTION DETAILLEE DE L'INVENTION

La présente invention concerne un dispositif 1 permettant l'analyse temporelle de la régularité et de la symétrie d'une séquence de N cycles de marche ou de course d'une personne, sans calcul de paramètres basés sur la considération globale des N cycles de marche ou de course, en comparant chaque cycle par rapport à chacun des autres cycles.

L'expression 'cycle de marche ou de course' correspond aux phases définies entre deux appuis du talon d'un même pied sur le sol lors de la marche ou d'une course de la personne et qui ont lieues à certains instants entre les deux appuis du talon. Elle est donc représentative du comportement du pied de la personne pendant un pas.

Comme il est connu de l'état de l'art, les différentes phases peuvent être définies en pourcentage, 0 % correspondant au premier appui talon et 100 % au deuxième.

Le cycle peut être séparé en deux phases : la phase d'appui (0 à 60%) où le pied est en contact avec le sol, et la phase oscillante ou pendulaire (60% à 100%) où le talon est en l'air.

Cette expression définit tous les styles de marche et les styles de course ; par exemple le piétinement doit être considéré comme faisant partie de la catégorie définie par l'expression 'cycle de marche'.

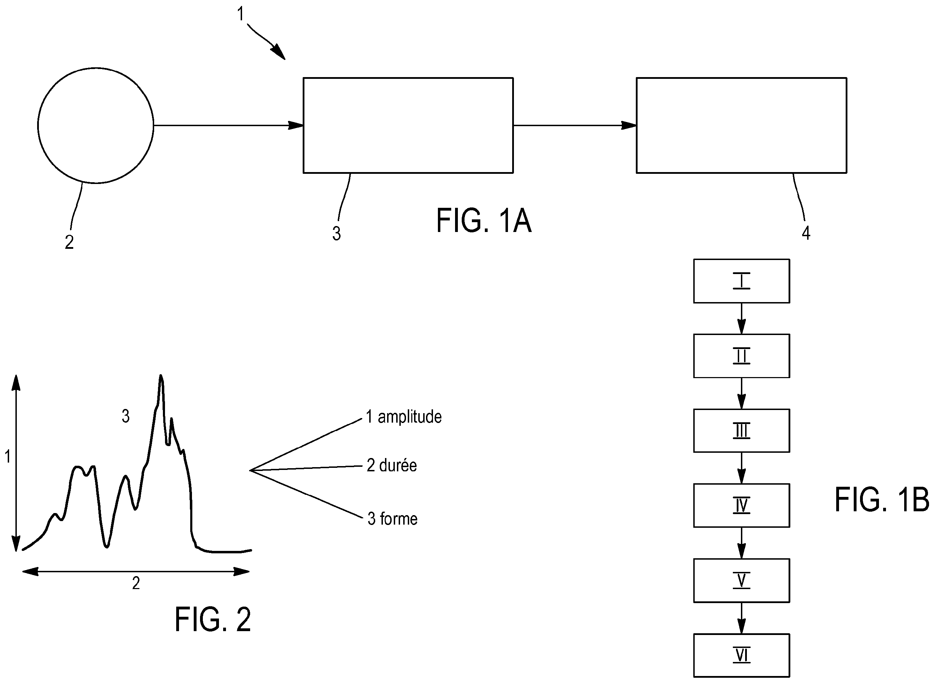

Le dispositif 1 illustré sur la figure 1a comporte des capteurs de mesure 2 de signaux bruts i d'une grandeur physique d'un segment anatomique, mesurée lors d'une séquence de N cycles de marche ou de course d'au moins un pied de la personne.

Les capteurs de mesure 2 peuvent être tout capteur, par exemple tel qu' : un accéléromètre, un gyroscope, une électromyographie, des semelles de pression ou des traceurs infrarouges/dispositifs d'acquisition vidéo ou IR.

Ils peuvent être placés par exemple sur le pied, la cheville, la ceinture, poignet, tête.

La grandeur physique mesurée peut, par exemple, être la norme de l'accélération, la norme de l'accélération sans la composante de gravité terrestre, la vitesse, la vitesse angulaire, le déplacement, la position etc...

Par exemple, les grandeurs issues de capteurs inertiels peuvent être l'accélération linéaire ou la vitesse angulaire selon un des 3 axes de l'espace. Avec d'autres capteurs, d'autres grandeurs peuvent être la position du pied dans l'espace avec la stéréophotogrammétrie ou la pression au sol avec des semelles de force à capteur de pression.

Comme représenté sur la figure 1a, le dispositif 1 comporte une unité de traitement et de calcul 3 agencée pour :

- traiter et séparer les données brutes en signaux temporels distincts Ci, chaque signal temporel Ci étant une série de points de la grandeur physique mesurée fonction du temps et présentant une forme, une amplitude et une durée donnée, la série Ci étant associée à un seul cycle donné i de la personne parmi les N cycles de marche comme représenté sur la figure 2 ;

- calculer au moins un coefficient de similarité entre un signal Ci associé à un cycle i, avec un autre signal Cj associé à un autre cycle j, représentatif de la similarité entre les deux signaux Ci et Cj ;

- ranger dans une matrice carrée M(i,j) indexée en ligne i et en colonne j, et indiquant pour chaque cellule (i, j), la valeur du coefficient de similarité entre les cycles i et j.

Lors de ces calculs et du rangement des valeurs déterminées dans la matrice M(i,j), les entiers naturels avec i et j varient de 1 à N, et les N cycles de marche étudiés sont ordonnés pour le pied donné, chronologiquement suivant leur ordre dans la séquence de la marche.

Ainsi par exemple,

- le cycle 1 est comparé au cycle 2 ( m[1,2] ou m[2,1] ) , cycle 3( m[1,3] ou m[3,1]) , cycle 4 ( m[1,4] ou m [4,1]) , etc...
- le cycle 2 est comparé au cycle 1 ( m[2,1] ou m[1,2] ) , cycle 3( m[3,1] ou m[1,3]) , cycle 4 ( m[2,4] ou m[4,2]) , etc...

Et ainsi de suite.

La matrice visualise par construction le cas aussi où le cycle 1 ( m[1,1] ),le cycle 2 etc.. sont comparés à eux-mêmes.

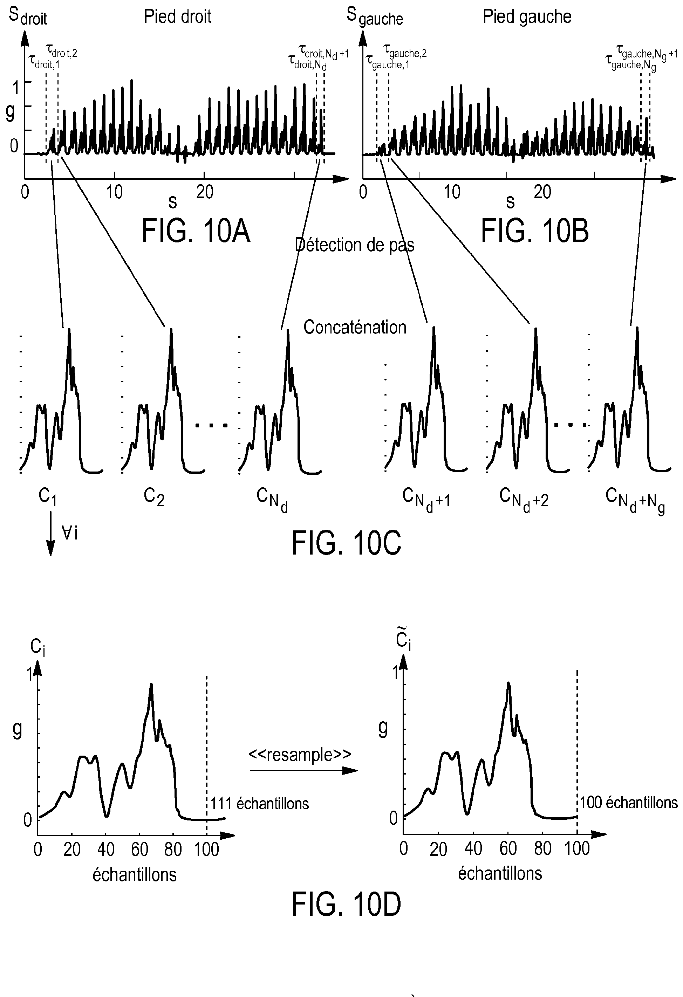

Le traitement (« compend resampling ») et la séparation des données brutes est illustré sur les figures 10A à 10D, et les signaux temporels distincts Ci, après traitement - dessus sont nommés $\tilde{C}_i$ sur la figure 10D (ce qui correspond à la terminologie C'i dans le texte).

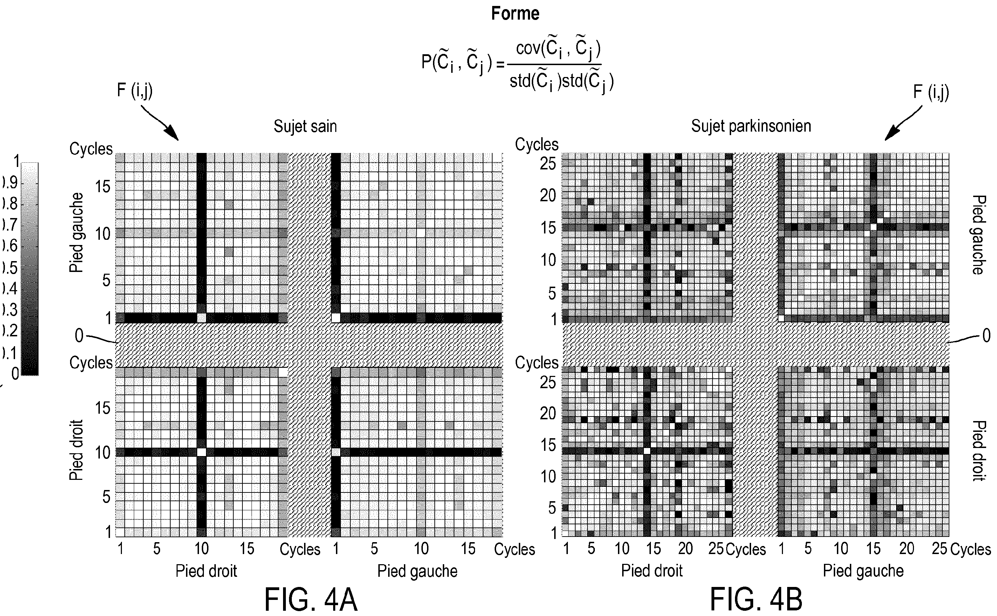

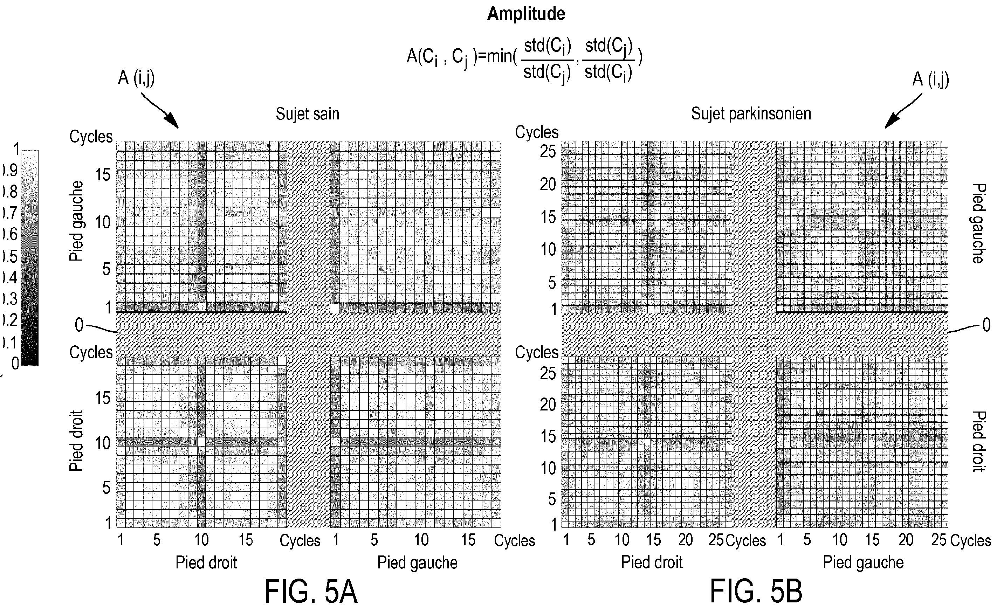

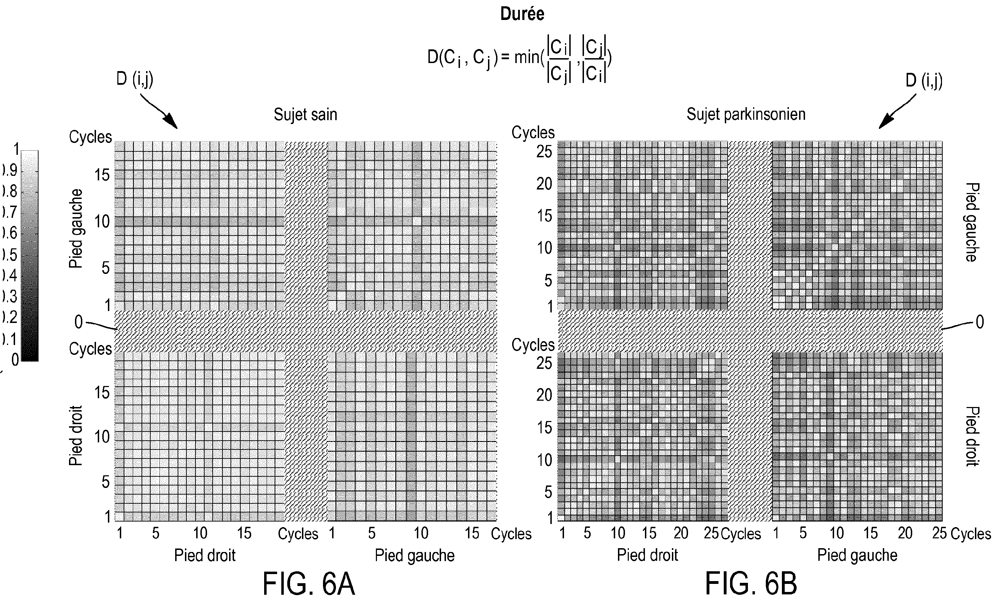

Le coefficient de similarité peut être : un coefficient de similarité de la forme fij des deux signaux Ci et Cj (comme représenté sur les figures 4a et 4b), un coefficient de similarité de l'amplitude aij des deux signaux Ci et Cj (comme représenté sur les figures 5a et 5b), ou un coefficient de similarité de la durée dij des deux signaux Ci et Cj (comme représenté sur les figures 6a et 6b).

Un synonyme pour 'coefficient' est 'distance algébrique' au sens mathématique du terme par exemple, et pour 'similarité' est ressemblance.

L'expression 'de similarité' implique la symétrie des coefficients et donc que fij=fji, aij=aji et dij=dji.

Le coefficient de similarité peut être considéré comme étant un coefficient de corrélation dans un certain nombre de cas de figures.

D'autres coefficients de similarité peuvent être utilisés en fonction de l'aspect de la forme que l'on veut comparer entre deux signaux : le Dynamic Time Wraping (DTW), le coefficient de Spearman, les distances euclidiennes (L1, L2 et L∞).

Ainsi, avec un coefficient de similarité qui varie dans un intervalle [a ; b],

- si deux signaux (ou les deux cycles de marche ou de course) sont identiques, le coefficient peut être égal à b, et
- si deux signaux (ou les deux cycles de marche ou de course) n'ont aucune ressemblance, le coefficient peut être égal à a,

la similarité entre les signaux augmentant linéairement entre a et b.

Avantageusement, l'unité de traitement et de calcul 3 est agencée pour :

- calculer plusieurs coefficients de similarité fij, aij, dij différents, chaque coefficient de similarité fij, aij, dij étant associé à la même échelle de couleurs, chaque couleur ayant la même signification de similarité quelle que soit le coefficient utilisé fij, aij, dij, comme illustré sur les figures 4a à 6b ;
- présenter les valeurs des coefficients de similarité fij, aij, dijj dans des matrices carrées différentes F(i,j), A(i,j) D(i,j), une matrice carrée par type de coefficient de similarité .

Le dispositif 1 présente aussi des moyens d'affichage 4 reliés à l'unité de traitement et affichant la matrice M(i,j) pour i et j variant de 1 à N, chaque valeur du coefficient de similarité étant représentée dans la matrice M(i,j) par une représentation visuelle graduée pour permettre de visualiser à l'œil nu la similarité entre les cycles de marche ou de course i et j.

Avantageusement, les coefficients de similarité (fij, dij, aij) sont choisis :

- pour qu'une fois calculées, toutes les valeurs des coefficients de similarité (fij, dij, aij) soient dans un même intervalle [a ; b] et
- pour que plus les valeurs des coefficients de similarité (fij, dij, aij) soient élevées et plus les signaux Ci, Cj soient similaires.

Avantageusement, cette représentation visuelle est graduée pour représenter la valeur du coefficient de similarité. En d'autres termes, il y a une correspondance entre l'échelle des valeurs de coefficient de similarité et celle des couleurs.

Cette correspondance peut être proportionnelle ou non.

De façon préférée, chaque valeur est représentée par une couleur située dans une échelle de couleurs graduées pour permettre de visualiser à l'œil nu la similarité entre les deux cycles de marche ou de course i et j (ce qui revient à étudier la similarité entre les deux signaux Ci et Cj).

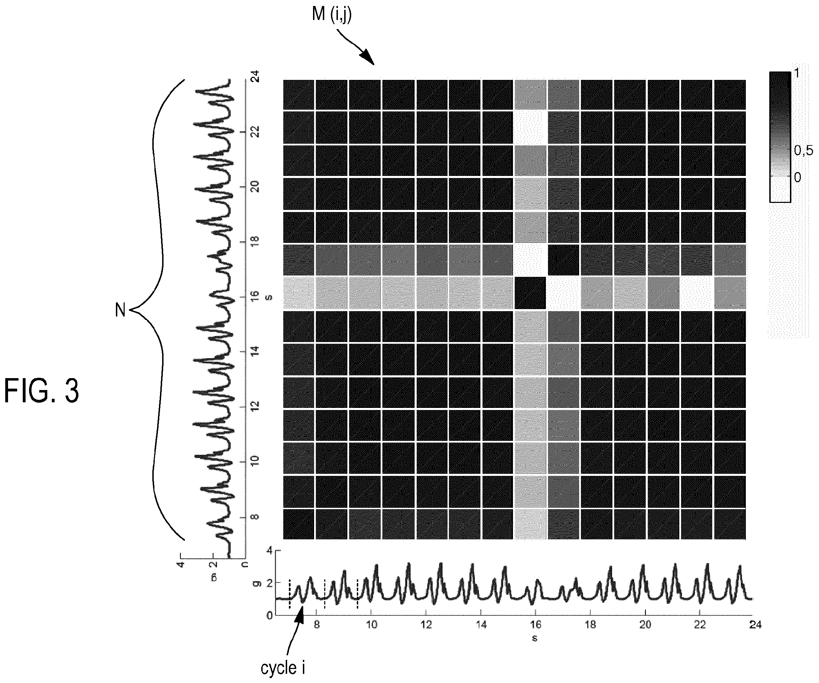

Avantageusement, l'échelle utilisée par les moyens d'affichage 4 est choisie sans seuils ou continue comme illustré sur les figures 3 à 6b.

Ici l'échelle est [0 ; 1] pour le coefficient de similarité de forme, le coefficient de similarité d'amplitude et le coefficient de similarité de durée permettant une comparaison rapide et aisée des différents coefficients de similarité entre eux.

Chaque case de la matrice peut porter une couleur correspondant au degré de similarité/ressemblance de deux pas entre eux.

Une couleur chaude (exemple rouge) est proche de 1, et traduit une grande ressemblance (similarité).

Une couleur froide (exemple bleu) est proche de 0, et traduit une faible ressemblance (similarité).

A noter que cette échelle est inversée entre la figure 3 d'un côté et les figures 4a à 6b de l'autre : sur la figure 3 des couleurs foncées indiquent une ressemblance, ce qui est le contraire sur les figures 4a à 6b ou des couleurs claires indiquent une ressemblance.

Les moyens d'affichage 4 affichent une représentation visuelle de la matrice de similarité telle que :

- chaque case représente un coefficient de similarité entre le signal Ci et Cj ;
- la valeur du coefficent de similarité (fij, aij, dij) est représentée dans une échelle de couleur graduée.

Dans un premier mode de réalisation illustré sur la figure 3, il est représenté le coefficient de similarité entre le signal Ci relatif à la marche ou à la course d'un seul pied (pied droit, ou respectivement pied gauche) de la personne, et le signal Cj relatif à la marche ou à la course du même pied, et nombre de cycles N=Nd (respectivement Ng) nombre de cycles du pied droit (respectivement nombres de cycles du pied gauche).

Dans un second mode de réalisation illustré sur les figures 4a à 6b, les moyens d'affichage 4 affichent:

- les coefficients de similarité (fij, aij, dij) calculés pour un seul pied 3 ;
- également le coefficient de similarité (fij, aij, dij) entre le signal Ci relatif à la marche ou à la course du pied droit de la personne, et le signal Cj relatif à la marche ou à la course du pied gauche de la personne, et N=Nd +Ng.

Dans ce cas, tous les cycles étudiés du pied droit, puis tous les cycles étudiés du pied gauche (ou inversement) sont ordonnés chronologiquement, en abscisse et en ordonnée de la matrice, comme illustré sur les figures 4a à 6b. Ils sont séparés ici dans la représentation par une zone O, ce qui donne 4 sous-carrés.

Ainsi, la matrice de similarité :

- « pied droit/pied droit » est représentée par le sous-carré en bas à gauche ;
- « pied gauche/pied gauche » est représentée par le sous-carré en haut à droite ;
- « pied gauche/pied droit » est représentée par le sous-carré en bas à droite.

Lorsque le coefficient de similarité est un coefficient de forme fij, l'unité de traitement et de calcul 3 est agencée pour :

- normaliser en durée chaque signal temporel Ci avec chaque signal temporel Cj pour que les deux signaux Ci et Cj aient la même durée ;
- normaliser en amplitude chaque signal temporel Ci ;
- calculer un coefficient de similarité de forme fij entre chaque signal normalisé C'i, avec un autre signal normalisé C'j, et ce pour tous les cycles de marche.

Dit autrement, pour étudier la ressemblance de la forme des cycles entre eux, il faut s'affranchir de leur différence d'amplitude et de leur différence de durée.

Par exemple, la normalisation en durée est réalisée par ré-échantillonage linéaire, ou par DTW, comme illustré sur la figure 10c.

Par exemple, la normalisation en amplitude est réalisée en divisant le Signal Ci par l'écart type ou par valeur efficace (« Root Mean Square »).

Dans une première réalisation, le coefficient de similarité de forme fij peut être le coefficient de Pearson, comme illustré sur les figures 4a et 4b, les valeurs étant ramenées à 0 en cas de valeurs du coefficient de Pearson inférieures à zéro.

D'autres distances mathématiques sont envisageables telles que la distance de corrélation de Spearman ou la technique du « dynamique time wraping ».

Si l'on s'intéresse uniquement à la forme, il est important de s'affranchir de l'amplitude et de la durée. Pour cela, il est re-normalisé le signal en ré-échantillonant le signal à 100 échantillons (normalisation en durée) et divisant le signal par l'écart-type du cycle (normalisation en amplitude). La distance de corrélation de Pearson conserve la ligne temporelle et compare strictement la forme des pas. La distance de corrélation de Spearman et le « dynamic time wraping » déforment la ligne temporelle et indique s'il existe une déformation possible pour que la forme de deux cycles se ressemble ou si les formes sont vraiment différentes.

Dans une deuxième réalisation, le coefficient de similarité peut être un coefficient de similarité de durée dij.

Dans ce cas, comme illustré sur les figures 5a et 5b, l'unité de traitement et de calcul 3 est agencée par exemple pour :

- calculer une durée Di, Dj du signal Ci et de la durée du signal Cj,
- calculer le coefficient de similarité de durée dij qui est le rapport de la plus petite des deux durées Di, Dj divisée par la plus grande durée Di, Dj, afin de ramener la valeur des coefficients entre 0 et 1.

La durée du signal Ci est égal au nombre d'échantillons, chaque échantillon étant pris de façon régulière à une fréquence donnée.

Dans une troisième réalisation, le coefficient peut être un coefficient de similarité d'amplitude.

Dans ce cas, comme illustré sur les figures 6a et 6b, l'unité de traitement et de calcul 3 est agencée par exemple pour :

- calculer une amplitude Ai, Aj du signal Ci et du signal Cj,
- calculer le coefficient de similarité d'amplitude aij qui est le rapport de la plus petite amplitude Ai, Aj divisée par la plus grande amplitude Ai, Aj afin de ramener la valeur des coefficients entre 0 et 1.

L'amplitude Ai et Aj est par exemple l'écart type ou la valeur efficace (« Root Mean Square »).

Une fois ces calculs effectués pour trouver les valeurs des coefficients de similarité, l'unité de traitement et de calcul 3 est agencée pour calculer :

- des paramètres permettant d'évaluer la régularité de la marche ;
- des paramètres permettant d'évaluer la symétrie de la marche ;
- le nombre de cycles nécessaires à établir une séquence ou une sous séquence de marche.

En particulier :

- le paramètre évaluant la régularité de la marche : la moyenne de toutes les cellules (i, j) de la matrice M(i,j) (A(i, j) ; D(i,j) ; F(i,j) ) en excluant les cellules i=j,
- le paramètre évaluant la régularité de la marche : la déviation standard de toutes les cellules (i, j) de la matrice M(i,j) en excluant les cellules i=j,
- le paramètre évaluant la symétrie de la marche : la moyenne des coefficients des cellules avec i allant de 1 à Nd (ou de 1 à Ng), et j allant de Nd+1 à Nd+Ng (j allant de Ng+1 à Nd+Ng).

La présente invention concerne aussi un procédé illustré sur la figure 2 et utilisant le dispositif 1 ci-avant décrit.

Le procédé, illustré sur la figure 2, comporte :

- une étape de détection (i) des signaux temporels bruts ;
- une étape de traitement et de séparation (ii) des signaux temporels bruts en signaux temporels distincts Ci;
- une étape de calcul (iii) de coefficient de similarité ;
- une étape de rangement (iv) dans une matrice M(i,j), la valeur du coefficient de similarité à la ligne i et à la colonne j ;

avec i et j entiers naturels variant de 1 à N, les N cycles de marche ou de course étant ordonnés chronologiquement;

- une étape d'affichage (v) affichant la matrice M(i,j) ;
- une étape de visualisation (vi) par un opérateur.

L'étape de visualisation par l'opérateur permet de déterminer :

- le nombre de cycles nécessaires à la réalisation d'une séquence de marche ou de course ou d'une sous-séquence de marche, la sous-séquence de marche ou de course étant: un demi-tour, l'initiation de la marche ou de la course, une accélération, une décélération, un régime établi, l'arrêt, un virage ;
- le nombre de cellules ayant une couleur associée à une valeur faible de coefficient ;
- si un cycle présente une ou plusieurs cellules ayant une couleur associée à une valeur faible ou élevée de coefficient ;
- si les deux cycles de marche consécutifs présentent une ou plusieurs cellules ayant une couleur associée à une valeur faible ou élevée de coefficient;
- repérer les changements de régime de pas au cours de l'exercice par des hétérogénéités de couleur ;
- comparer la couleur des cellules pied-droit-pied droit avec les cellules pied droit-pied gauche.

Il peut être prévu que l'unité de traitement et de calcul 3 compte le nombre de cycles ci-dessus définis, ou ne fait apparaitre que certaines valeurs de coefficients de similarité entre les cycles au vu de valeurs de seuil prédéfinies.

**DESCRIPTION DU PROTOCOLE DE REALISATION D'UN EXERCICE DE MARCHE ET DESCRIPTION DE LA COHORTE DE PARKINSONIENS D'APRES AVC 2.**

<u>Etape 1 : Acquisition des données</u>

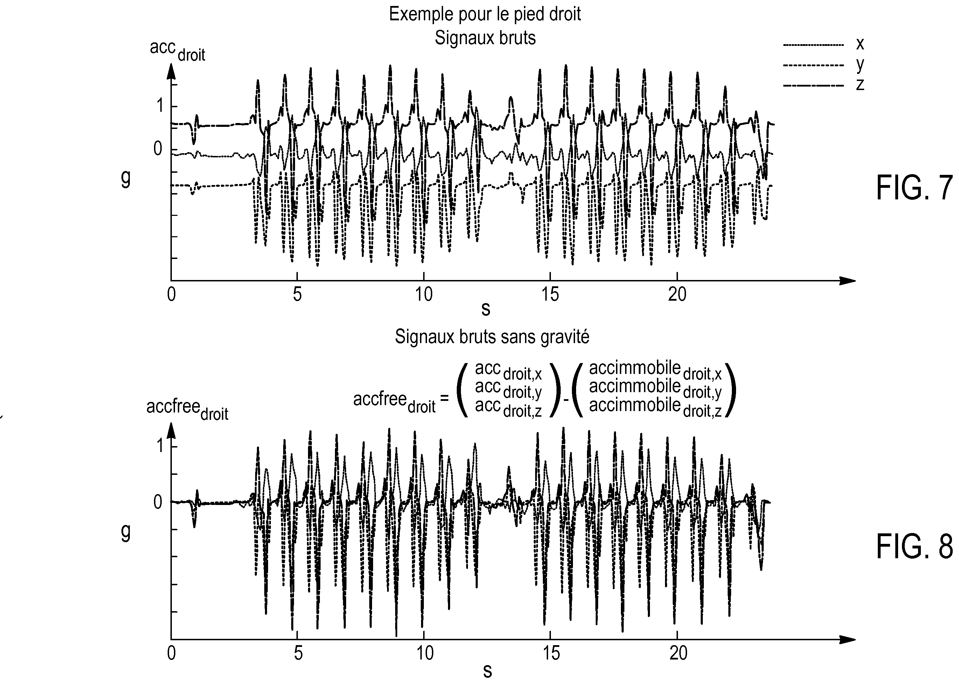

Un sujet portant un accéléromètre triaxial sur la face dorsale de chaque pied réalise un exercice de marche de 10 mètres aller-retour départ arrêté. Chacun des capteurs enregistre un signal temporel en 3 dimensions (selon les trois axes x, y et z). On appelle respectivement $\boldsymbol{acc}_{droit}$ et $\boldsymbol{acc}_{gauche}$ les signaux pour le pied droit et le pied gauche, comme représenté sur les courbes de la figure 7.

Etape 2 : Repérage des débuts des cycles de marche

On repère (manuellement ou automatiquement) dans les signaux, les temps correspondant aux débuts des cycles de marche. Ces instants, selon la définition de Mariani et al. (2013), correspondent aux « heel-strikes », c'est-à-dire aux instants où le talon touche le sol. On définit deux ensembles $\tau_{droit}$ et $\tau_{gauche}$ correspondant respectivement à l'ensemble des heel-strike pour le pied droit et le pied gauche (figures 10A et 10B). On appelle $N_d + 1$ et $N_g + 1$ respectivement le nombre de heel-strike repérés pour le pied droit et pour le pied gauche. Le cycle de marche i du pied droit est défini comme la partie du signal commençant à l'instant $\tau_{droit,i}$ et finissant au début du cycle de marche suivant $\tau_{droit,i+1}$, comme le montrent les figures 10A et 10B. Ainsi, $N_d$ et $N_g$ sont les nombres de cycle de marche du pied droit et du pied gauche respectivement.

Etape 3 : Retrait de la gravité

Au début du protocole, ou dans une étape préliminaire, on a demandé au sujet de rester debout et immobile. On a enregistré la valeur (constante) des accélérations durant cette phase et on l'a stocké dans un vecteur $\begin{pmatrix} \boldsymbol{accimmobile}_{droit,x} \\ \boldsymbol{accimmobile}_{droit,y} \\ \boldsymbol{accimmobile}_{droit,z} \end{pmatrix}$ Comme représenté sur les courbes de la figure 8, il est enlevé la gravité selon le procédé suivant :

$$\boldsymbol{accfree}_{droit} = \begin{pmatrix} \boldsymbol{acc}_{droit,x} \\ \boldsymbol{acc}_{droit,y} \\ \boldsymbol{acc}_{droit,z} \end{pmatrix} - \begin{pmatrix} \boldsymbol{accimmobile}_{droit,x} \\ \boldsymbol{accimmobile}_{droit,y} \\ \boldsymbol{accimmobile}_{droit,z} \end{pmatrix}$$

On réitère le même procédé pour le pied gauche.

Etape 4 : Calcul de la norme

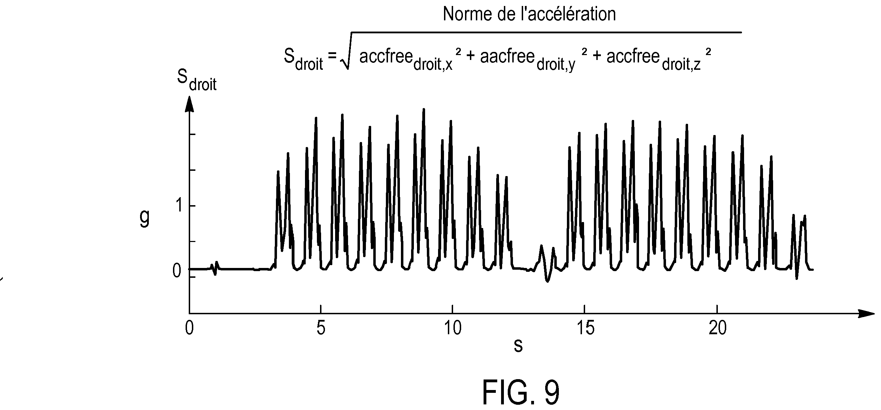

Comme représenté sur la courbe de la figure 9, il est calculé la norme du vecteur accélération à chaque instant t :

$$\boldsymbol{s}_{droit}[t] = \sqrt{\boldsymbol{accfree}_{droit,x}[t]^2 + \boldsymbol{accfree}_{droit,y}[t]^2 + \boldsymbol{accfree}_{droit,z}[t]^2}$$

de même pour le pied gauche.

Cette fusion des trois axes de l'accélération permet de se rendre indépendant de la position du capteur qui est une source d'imprécision de mesure importante dans la mesure par accéléromètre.

Etape 5 : Création des cycles de marche

Comme représenté sur la courbe des figures 10C à 10D, on note l'ensemble des cycles de marche de l'exercice de la manière suivante :

Pour

$$\forall i \in [\![1, N_d + N_g]\!] \begin{cases} \boldsymbol{c}_i = \boldsymbol{s}_{droit}\left[\boldsymbol{\tau}_{droit,i}, \boldsymbol{\tau}_{droit,i+1}\right] si\ 1 \leq i \leq N_d \\ \boldsymbol{c}_i = \boldsymbol{s}_{gauche}\left[\boldsymbol{\tau}_{gauche,i-N_d}, \boldsymbol{\tau}_{gauche,i-N_d+1}\right] si\ N_d + 1 \leq i \leq N_d + N_g \end{cases}$$

On obtient $\{c_1, c_2, \ldots, c_{N_d}, c_{N_d+1}, c_{N_d+2}, \ldots, c_{N_d+N_g}\}$ l'ensemble des cycles de marche d'un exercice de marche avec $\{c_1, c_1, \ldots, c_{N_d}\}$ les cycles du pied droit et $\{c_{N_d+1}, c_{N_d+2}, \ldots, c_{N_d+N_g}\}$ les cycles du pied gauche.

Etape 6 : Calcul des métriques

De toutes les étapes présentes, il est déterminé un signal temporel Ci comportant une série de points de la grandeur physique mesurée fonction du temps.

Chaque signal temporel Ci présente une forme, une amplitude et une durée donnée, la série Ci étant associée un cycle donné i, comme représenté sur la figure 2.

**6.1 Quelques notations préliminaires**

Etant donné un vecteur $x$ composé de N échantillons$\{x_1, x_2, \dots, x_N\}$, on définit les quantités suivantes :

Moyenne :

$$\bar{\boldsymbol{x}} = \frac{1}{N}\sum_{i=1}^{N} x_i$$

Ecart type :

$$std(\boldsymbol{x}) = \frac{1}{N}\sum_{i=1}^{N}(x_i - \bar{x})^2$$

Etant donnés deux vecteurs ***x*** et ***y***, on définit :
Covariance :

$$cov(\boldsymbol{x},\boldsymbol{y}) = \frac{1}{N}\sum_{i=1}^{N}(x_i - \bar{x}) \quad (y_i - \bar{y})$$

**6.2 Métrique durée**

On définit la métrique durée par :

$$\forall i, j \in [\![1, N_d + N_g]\!]^2 \; D(\boldsymbol{c}_i, \boldsymbol{c}_j) = \min(\frac{|\boldsymbol{c}_i|}{|\boldsymbol{c}_j|}, \frac{|\boldsymbol{c}_j|}{|\boldsymbol{c}_i|})$$

ou $|\boldsymbol{c}_j|$ est le nombre d'éléments du cycle *j*.

Il s'agit du rapport des durées des cycles en mettant toujours le cycle le plus long au dénominateur.

**6.3 Métrique amplitude**

On définit la métrique amplitude par :

$$\forall i, j \in [\![1, N_d + N_g]\!]^2 \; A(\boldsymbol{c}_i, \boldsymbol{c}_j) = \min(\frac{std(\boldsymbol{c}_i)}{std(\boldsymbol{c}_j)}, \frac{std(\boldsymbol{c}_j)}{std(\boldsymbol{c}_i)})$$

Il s'agit du rapport des déviations standards en mettant toujours le cycle ayant l'amplitude la plus grande au dénominateur.

**6.4 Métrique forme**

A ce stade, les cycles n'ont pas tous la même longueur. On normalise la longueur de chaque cycle à 100 échantillons en utilisant la fonction « resample » de Matlab ® ("MATLAB 2014a, The MathWorks, Natick, 2014."), comme représenté sur les figures 10D. On appelle $\widetilde{\boldsymbol{c}_i}$ la version renormalisée de $\boldsymbol{c}_i$

On définit enfin la métrique forme par :

$$F(\boldsymbol{c}_i, \boldsymbol{c}_j) = \max(0, P(\widetilde{\boldsymbol{c}_i}, \widetilde{\boldsymbol{c}_j})$$

Où

$$\forall i, j \in [\![1, N_d + N_g]\!]^2 \; P(\widetilde{\boldsymbol{c}_i}, \widetilde{\boldsymbol{c}_j}) = \frac{cov(\widetilde{\boldsymbol{c}_i}, \widetilde{\boldsymbol{c}_j})}{std(\widetilde{\boldsymbol{c}_i})std(\widetilde{\boldsymbol{c}_j})}$$

Etape 7 : Construction des matrices de visualisation

Ainsi on obtient 3 matrices pour un exercice de marche

$$[D(c_i, c_j)]_{\substack{i=1..N_d+N_g \\ j=1..N_d+N_g}} \quad [A(c_i, c_j)]_{\substack{i=1..N_d+N_g \\ j=1..N_d+N_g}} \quad [F(c_i, c_j)]_{\substack{i=1..N_d+N_g \\ j=1..N_d+N_g}}$$

On a la forme suivante :

$$\begin{bmatrix} Comparaison\ droite\ gauche & Comparaison\ gauche \\ Comparaison\ droit & Comparaison\ droite\ gauche \end{bmatrix}$$

**DESCRIPTION DETAILLEE DES MATRICES**

Il est ici décrit les figures 4A à 6B.

Les figures présentées dans ce document ont une valeur illustrative. D'après la construction du « locogramme », la taille ne diffère pas entre la forme, l'amplitude et la durée. Les différences de nombre de carrés observées ici entre les figures 4A et 6B sont liées à un défaut de mise en page.

Le sujet sain a réalisé un total de 38 cycles contre 54 pour le sujet parkinsonien pour la même distance ce qui traduit une marche à petits pas pathologique (figure 4A et 4B). Dans la matrice de forme du sujet sain, les carreaux homogènes de couleur claire traduisent une marche régulière (figure 4A). On observe un régime établi de marche atteint en 1 cycle puisque seul le cycle n°1 du pied droit est très différent des autres.

Pour le sujet parkinsonien, l'hétérogénéité de la matrice de forme traduit une marche irrégulière (figure 4B). Le régime stationnaire est atteint en 2 cycles (cycle n°1 du pied gauche et cycle n°1 du pied droit).

Les plages d'homogénéité le long de la diagonale du « locogramme » sont des signes de marche de qualité. En effet, cela signifie que tous les cycles de cette plage d'homogénéité se ressemblent entre eux et sont réguliers.

Sur la figure 4A (sujet sain), ces plages sont longues et omniprésentes. On en distingue deux pour chaque pied qui correspondent à l'aller et au retour de l'exercice de marche. L'aller et le retour pour chaque pied sont séparés par un cycle noir (croix centrale dans les carrés en bas à gauche et en haut à droite : cycle n° 10 pied droit par exemple) qui correspond au cycle de demi-tour et qui de manière normale ne ressemble à aucun autre cycle.

Sur la figure 4B (sujet pathologique) les plages d'homogénéité sont plus courtes. On repère plusieurs plages homogènes : cycles n° 2 à 6 et cycles n° 20 à 25 pour le pied droit, cycles n° 2 à 7, cycles n° 10 à 14 et cycles n° 15 à 26 pour le pied gauche. Il est à noter que ces plages d'homogénéité de qualité relative. En effet, elles sont homogènes comparées au reste du «locogramme» de la figure 4B mais relativement hétérogènes comparées au «locogramme» de la figure 4A. Sur la figure 4B, ces plages sont en début de marche : le sujet n'est pas fatigué et peut réaliser une marche de qualité. Ces plages sont également en fin d'exercice pouvant traduire un temps important nécessaire à l'obtention d'un régime établi de marche. Au milieu de l'exercice, la marche est dégradée en témoigne l'absence de plages homogènes.

Bien plus, on observe des cycles erratiques traduisant des irrégularités bien localisées dans le temps ('freezing', trébuchement) : cycle n° 19 pied droit, cycles n° 8 et n° 9 pied gauche par exemple. Enfin, on observe également des irrégularités plus diffusent dans le temps prenant la forme de plages hétérogènes : cycles n° 15 à 20 pied droit et cycles n° 15 à 20 pied gauche par exemple. Il est à noter que ces deux plages hétérogènes suivent immédiatement le demi-tour. Ceci traduit peut être une difficulté à réaliser le demi-tour et à reprendre une marche de qualité après celui-ci. Il n'y a pas de plages hétérogènes et ni de cycles erratiques observables sur la figure 4A.

Les demi-tours sont également révélateurs de la qualité de la locomotion. Sur la figure 4A (sujet sain), le demi-tour est composé de seulement de 1 cycle de manière physiologique : cycle n° 10 pied droit. Sur la figure 4B (sujet pathologique), le demi-tour est composé de 2 cycles : cycle n° 14 pied droit et cycle n° 15 pied gauche. On constate que le demi-tour sur la figure 4B a une répercussion sur les cycles alentours (cycles n° 14 à 17 pied gauche sont plus sombres) tandis qu'on observe pas de telle répercussion dans la figure 4A.

Dans la matrice d'amplitude du sujet sain, la croissance, puis la décroissance de la similarité de l'amplitude de l'accélération entre les cycles traduit la mise en place de la vitesse de croisière (figure 5A). Cette croissance et cette décroissance sont moins visibles pour le sujet parkinsonien (figure 5B).

Dans la matrice de durée du sujet témoin, la couleur globale plus claire que celle du sujet parkinsonien traduit une plus grande ressemblance entre les cycles de marches en termes de durée (figure 6A et 6B). L'homogénéité de la couleur traduit une moins grande variabilité entre les cycles de marches en termes de durée.

## ANALYSE NUMERIQUE A PARTIR DU « LOCOGRAMME » SUR 40 SUJETS

Nous calculons la déviation standard (DS) de la durée des cycles de marches sur la marche établie (ensemble des cycles de marche sans les 3 premiers cycles, les 3 cycles précédent le demi-tour, les cycles du demi-tour, les trois cycles suivants le demi-tour et les trois derniers cycles de l'exercice) :

$$P0 = DS|\boldsymbol{c}_i|_{i=marche\ \text{é}tablie}$$

où $|\boldsymbol{c}_i|$ est le nombre d'échantillons de $\boldsymbol{c}_i$.

Nous calculons la moyenne du « locogramme » sur la marche établie :

$$P1 = moyenne\left[F\left(c_i, c_j\right)\right]_{\substack{i=marche\ \text{é}tablie \\ j=marche\ \text{é}tablie}}$$

Nous calculons la déviation standard du « locogramme » sur la marche établie :

$$P2 = DS\left[F\left(c_i, c_j\right)\right]_{\substack{i=marche\ \text{é}tablie \\ j=marche\ \text{é}tablie}}$$

Nous calculons le nombre de clusters hiérarchiques sur le « locogramme » obtenu avec la même règle d'arrêt :

$$P3 = cluster\left[F\left(c_i, c_j\right)\right]_{\substack{i=1..N_r+N_l \\ j=1..N_r+N_l}}$$

Les résultats comparant les paramètres {P1, P2, P3} pour deux groupes de sujets sains (jeune et âgés) et des patients atteints de la maladie de Parkinson sont rapportés dans le tableau 1. Les résultats comparant la corrélation des paramètres {P1, P2, P3} avec la sévérité de l'atteinte clinique des patients atteints de la maladie de Parkinson (évaluée avec le score UPDRS III) et la qualité de la marche (évaluée avec la vitesse de marche et P0) sont représentés dans le tableau 2.

Les résultats montrent que le « locogramme » des patients atteints de la maladie de parkinson est significativement plus dégradé que celui des sujets sains âgés et des sujets jeunes sur les 3 paramètres {P1, P2, P3} issus du « locogramme » (Tableau 1).

D'autre part, il y a une corrélation entre la clinique (score UPDRS III) et la qualité de la marche évaluée par le «locogramme» selon les trois paramètres {P1, P2, P3} issus du «locogramme» (Tableau 2).

Enfin, il y a une corrélation entre la qualité de la marche évaluée avec les paramètres de l'état de l'art (vitesse de marche et P0) et la qualité de la marche évaluée par le «locogramme» selon les trois paramètres {P1, P2, P3} (Tableau 2).

Donc, sur un ensemble de 40 sujets, le «locogramme» permet d'évaluer numériquement la qualité de la marche d'un patient atteint de la maladie de Parkinson (en plus du point de vue visuel).

## AVANTAGE DES MATRICES

La matrice de visualisation affichée sur les figures 4A et 6B calculée sur un exercice de marche de 10 mètre aller/retour départ arrêté donne accès aux paramètres de marche suivants :

- Nombre de cycles nécessaires à la réalisation de l'exercice ;
- Nombre de cycles nécessaires pour le demi-tour ;
- Nombre de cycles nécessaires à l'initiation de la marche.

La matrice de visualisation présente ces paramètres de manière plus intuitive que sous forme de liste. De plus, pour les deux derniers paramètres, la définition dans la littérature repose sur des seuils fixés expérimentalement. L'intérêt d'une représentation globale plutôt que d'un paramètre est d'éviter les erreurs dues à un seuil arbitraire.

La forme de la matrice de visualisation permet de répondre aux questions suivantes :

- La marche est-elle globalement régulière ?
- Le sujet a-t- il atteint un régime de marche établi ? A quel moment ?
- Y a-t- il plusieurs régimes de marche établie différents ?
- Y a-t- il des cycles de marche erratiques («freezing» en anglais) ? A quel moment ?

La matrice de visualisation permet d'avoir une idée du déroulé de l'exercice de marche contrairement aux paramètres qui n'ont pas de valeur temporelle. La matrice de visualisation donne accès à la notion de régime de marche qui est une notion pour laquelle nous ne disposons pas d'outils d'exploration mise à part le tracé de paramètre chronologiquement cycle de marche après cycle de marche.

Chaque sujet possède un style particulier de marche à l'origine d'une grande variabilité interindividuelle des paramètres. Ce style se traduit dans les signaux par une signature très reproductible d'un cycle de marche sur l'autre chez le sujet sain. Les paramètres ne sont pas un outil adapté à la description de cette signature. La matrice de visualisation, avec un choix adapté de distance (voir la rubrique « description de l'invention ») compare la forme des pas et évalue la ressemblance de la signature d'un cycle vis-à-vis des autres cycles de marche. Ainsi, il évalue la qualité de la reproductibilité de la marche et gomme le style personnel car dans la distance entre de deux cycles d'une même personne, les variabilités individuelles s'annulent. La matrice de visualisation donne pour chaque sujet un tableau de cellules portant un chiffre compris entre 0 et 1. Ceci permet de comparer les matrices de visualisation entre eux de manière fiable.

La matrice de visualisation donne accès à de nouveaux paramètres de marche inédits comme :

- Le nombre de clusters avec la même règle d'arrêt : un sujet pathologique en aura d'avantage.
- La variabilité de la marche calculée avec la moyenne et la déviation standard de tous les carrés du tableau en conservant ou non les pas de demi-tours ou d'initiation de la marche.

Ceci fait de la matrice de visualisation un outil utile à la mesure de la marche longue qui montrera si un sujet utilise deux régimes de marche différents qui est un indicateur clinique intéressant (réveil de la douleur dans l'arthrose ou «freezing» dans la maladie de Parkinson par exemple).

La marche est une activité pseudopériodique qui se subdivise naturellement en cycles modulés par des changements de rythme physiologiques dans la marche (i.e., l'initiation, le demi-tour et l'arrêt) ou des irrégularités pathologiques dans la marche établie. Sur un exercice de marche, il existe des cycles de marche de différente nature comme les cycle d'initiation de la marche (les 4 premiers cycles), les cycles de marche établie, les cycles de préparation de demi-tour, les cycle de demi-tour et les cycle d'arrêt de la marche. En se fondant sur cette propriété, la matrice de visualisation permet de représenter avec le même procédé tous les temps de la marche. Ceci fait de la matrice de visualisation un outil adapté à la représentation de la marche en ambulatoire qui implique toutes les phases de la marche.

L'application principale de la matrice de visualisation est la visualisation d'un exercice de marche. La matrice de visualisation peut être utilisée sur tout exercice de pas en incluant les 10 mètres aller/retour comme décrit ci-avant, les Time Up and Go test et aussi sur tapi de marche et la marche ambulatoire.

La matrice de visualisation est adaptée pour la visualisation d'un exercice de marche de 10 mètres aller/retour départ arrêté, mesuré avec la norme de l'accélération d'un accéléromètre placé sur la face dorsale du pied, en comparant les cycles de marche à l'aide de la distance de corrélation de Pearson. Ce protocole est adapté à la consultation clinique de routine et permet une représentation synthétique pour une visualisation directe en clinique.

**Tableau 1 : Comparaison inter-groupe de P1, P2 et P3 pour CJ, CA and MP.**

| Paramètres | | CJ | CA | MP | |
|---|---|---|---|---|---|
| P1 | Moyenne | 0,96 | 0,94 | 0,88 | I-III<br>II-III |
| | DS | 0,02 | 0,04 | 0,05 | |
| P2 | Moyenne | 0,03 | 0,04 | 0,09 | I-III<br>II-III |
| | DS | 0,02 | 0,04 | 0,07 | |
| P3 | Moyenne | 5,09 | 5,08 | 6,71 | I-III<br>II-III |
| | DS | 2,30 | 2,59 | 2,72 | |

(suite)

| Paramètres | | CJ | CA | MP | |
|---|---|---|---|---|---|
| Test statistique : ANOVA (analyse de variance). $^{\text{II-III}}$ = valeur-p < 0.05.<br>DS déviation standard<br>CJ (contrôles jeunes) groupe d'adultes jeunes (N=9 ; âge = moyenne 38,3 DS 8,2 ans)<br>CA (contrôles âgés) groupe d'adultes âgés (N=11 ; âge = moyenne 67,0 DS 10,1 ans)<br>MP (maladie de Parkinson) groupe de patients atteints de la maladie de Parkinson (N=20 ; âge = moyenne 74,7 DS 11,0 ans)<br>P1 paramètre moyenne du locogramme sur la marche établie (sans unité)<br>P2 paramètre déviation standard du locogramme sur la marche établie (sans unité)<br>P3 paramètre nombre de clusters hiérarchiques sur le locogramme obtenu avec la même règle d'arrêt (sans unité) | | | | | |

**Tableau 2 : Corrélation intra-groupe MP entre {P1, P2, P3} et {UPDRS III, vitesse de marche, P0}**

| Paramètre | | UPDRS III | Vitesse de marche | P0 |
|---|---|---|---|---|
| **P1** | Rho | -0,51 * | 0,67* | 0,30* |
| | Pval | 0,00 | 0,00 | 0,00 |
| **P2** | Rho | 0,39* | -0,44* | -0,16 |
| | Pval | 0,00 | 0,00 | 0,06 |
| **P3** | Rho | 0,23* | -0,47* | -0,27* |
| | Pval | 0,00 | 0,00 | 0,00 |
| Corrélations évaluées avec le coefficient de Pearson. * = valeur-p < 0.05.<br>Rho coefficient de corrélation<br>UPDRS III score clinique permettant de quantifier la sévérité de l'atteinte motrice de la maladie de Parkinson par le neurologue, en consultation.<br>MP (maladie de Parkinson) groupe de patients atteints de la maladie de Parkinson<br>P0 paramètre déviation standard de la durée des cycles de marches sur la marche établie<br>P1 paramètre moyenne du locogramme sur la marche établie<br>P2 paramètre déviation standard du locogramme sur la marche établie<br>P3 paramètre nombre de clusters hiérarchiques sur le locogramme obtenu avec la même règle d'arrêt | | | | |

## Revendications

**1.** Procédé permettant, par une représentation visuelle :

- l'analyse temporelle de la régularité et de la symétrie d'une séquence de N cycles de marche ou de course d'une même personne, chacun desdits cycles étant séparé en deux phases dont une phase d'appui où le pied de ladite personne est en contact avec le sol et une phase oscillante ou pendulaire où le talon de la personne est en l'air, en comparant chaque cycle pris individuellement avec chacun des autres cycles pris individuellement,
- et la détermination de la présence et du nombre de cycles erratiques ainsi que le nombre de cycles nécessaires à la réalisation des régimes de la marche ou de course, et à quel moment dans la séquence ces cycles erratiques et régimes de marche sont atteints,

comportant les étapes suivantes :

• une étape de mesure (i) de signaux temporels bruts inertiels ou relatifs à une grandeur physique de déplacement d'au moins un segment anatomique de la personne,
• une étape de traitement et de calcul, subdivisée en :

- une étape de traitement et de séparation (ii) des signaux temporels bruts en signaux temporels distincts Ci afin de décomposer la marche ou la course en cycles de marche ou de course,

chaque signal temporel distinct Ci étant une série temporelle de points de la grandeur physique mesurée et présentant une forme, une amplitude et une durée donnée, la série Ci étant associée à un cycle de marche ou de course donné i de la personne, la séparation des signaux temporels bruts en signaux temporels distincts Ci étant mise en œuvre au moyen d'un repérage, dans les signaux temporels bruts, des instants où le talon de la personne touche le sol ;

- une étape de calcul (iii) d'au moins un coefficient de similarité entre le signal Ci associé au cycle de marche ou de course i, avec un autre signal Cj associé à un cycle de marche ou de course j de la même personne,

le coefficient de similarité étant :

un coefficient de similarité de la forme fij des deux signaux Ci et Cj qui est indépendant de l'amplitude et de la durée, avec fij=fji, ou
un coefficient de similarité de l'amplitude aij des deux signaux Ci et Cj qui est indépendant de la forme et de la durée, avec aij=aji , ou
un coefficient de similarité de la durée dij des deux signaux Ci et Cj qui est indépendant de la forme et de la durée, avec dij=dji,

• une étape de rangement (iv) dans une matrice carrée M(i,j), la valeur du coefficient de similarité fij, aij ou dij à la ligne i et à la colonne j;
avec i et j entiers naturels variant de 1 à N, les N cycles de marche ou de course étant ordonnés chronologiquement suivant leur ordre dans la séquence de marche ou de course,
• une étape d'affichage (v) de la matrice carrée M(i,j) avec les valeurs du coefficient de similarité fij, aij ou dij représentées dans les cellules (i,j) de la matrice carrée M(i,j), par une représentation visuelle de la valeur du coefficient de similarité fij, aij ou dij située dans un intervalle entre deux valeurs extrêmes, de façon continue sans seuils,
pour permettre de déterminer visuellement et simultanément :

- la similarité entre l'ensemble des cycles de marche ou de course i et j de la même personne, en comparant chaque cycle pris individuellement avec chacun des autres cycles pris individuellement,
- le nombre de cycles erratiques et le nombre de cycles nécessaires à la réalisation des régimes de la séquence de marche ou de course, et à quel moment dans la séquence ces cycles erratiques et régimes sont atteints.

**2.** Procédé selon la revendication précédente, **caractérisé en ce que** dans l'étape de traitement et de calcul , il est déterminé le coefficient de similarité entre le signal Ci relatif à la marche ou à la course du pied droit (respectivement du pied gauche) de la personne, et le signal Cj relatif à la marche ou à la course du pied droit (respectivement du pied gauche) de la même personne, et **en ce que** N=Nd (respectivement Ng) qui est le nombre de cycles du pied droit (respectivement le nombre de cycles du pied gauche).

**3.** Procédé selon la revendication précédente 2, **caractérisé en ce que** dans l'étape de traitement et de calcul , il est déterminé également le coefficient de similarité entre le signal Ci relatif à la marche ou à la course du pied droit de la personne, et le signal Cj relatif à la marche ou à la course du pied gauche de la même personne, et **en ce que** le nombre de cycles N est égal à la somme du nombre de cycles du pied droit et du nombre de cycles du pied gauche : N=Nd +Ng.

**4.** Procédé selon l'une des revendications précédentes, **caractérisé en ce que** les régimes de marche ou de course déterminés sont : un ou plusieurs régimes établis, initiation de la séquence de marche ou de course, ou demi-tour.

**5.** Procédé selon l'une des revendications précédentes, **caractérisé en ce que**, dans l'étape d'affichage (v), il est représenté chaque valeur du coefficient de similarité par une couleur située dans une échelle continue graduée en correspondance avec l'échelle des coefficients de similarité.

**6.** Procédé selon la revendication précédente 5, **caractérisé en ce que** :

- dans l'étape de traitement et de calcul, il est calculé les trois coefficients de similarité différents fij, aij, dij, chaque coefficient de similarité fij, aij, dij étant associé à la même échelle de couleurs que celle des autres coefficients de similarité, chaque couleur ayant la même signification de similarité quel que soit le coefficient de similarité utilisé ;

- dans l'étape d'affichage (v), il est présenté les valeurs des coefficients de similarité fij, aij, dij dans des matrices carrées différentes (F(i,j), A(i,j),D(i,j));

les coefficients de similarité étant choisis :

- pour qu'une fois calculées, toutes les valeurs des coefficients de similarité fij, aij, dij soient dans un même intervalle [a ; b] et
- pour que plus les valeurs des coefficients de similarité fij, aij, dij soient élevées et plus les signaux Ci, Cj soient similaires.

**7.** Procédé selon l'une des revendications précédentes, **caractérisé en ce que** le coefficient de similarité est un coefficient de forme fij, et **en ce qu'**il est, dans l'étape de traitement et de calcul:

- normalisé en durée chaque signal temporel Ci avec chaque signal temporel Cj pour que les deux signaux Ci et Cj aient la même durée ;
- normalisé en amplitude chaque signal temporel Ci ;
- calculé un coefficient de similarité de forme fij entre chaque signal normalisé C'i, avec un autre signal normalisé C'j, et ce pour tous les cycles de marche ou de course.

**8.** Procédé selon la revendication précédente, **caractérisé en ce que** le coefficient de similarité de forme fij est le coefficient de Pearson, les valeurs du coefficient de similarité étant ramenées à 0 en cas de valeur du coefficient de Pearson inférieure à zéro.

**9.** Procédé selon l'une des revendications précédentes, **caractérisé en ce que** :

- le coefficient de similarité est un coefficient de similarité de durée dij,
- l'unité de traitement et de calcul (3) est agencée pour :
  - calculer une durée Di, Dj du signal Ci et de la durée du signal Cj,
  - calculer le coefficient de similarité de durée dij qui est le rapport de la plus petite des deux durées Di, Dj divisée par la plus grande durée Di, Dj.

**10.** Procédé selon l'une des revendications précédentes, **caractérisé en ce que** :

- le coefficient de similarité est un coefficient de similarité d'amplitude aij,
- et l'unité de traitement et de calcul (3) est agencée pour :
  - calculer une amplitude Ai, Aj du signal Ci et du signal Cj,
  - calculer le coefficient de similarité d'amplitude aij qui est le rapport de la plus petite amplitude Ai, Aj divisée par la plus grande amplitude Ai, Aj.

**11.** Procédé selon la revendication précédente, **caractérisé en ce que** l'amplitude Ai, Aj est l'écart type ou la valeur efficace.

**12.** Procédé selon l'une des revendications précédentes, **caractérisé en ce qu'**il est calculé :

- des paramètres permettant d'évaluer la régularité de la marche ou de la course ;
- des paramètres permettant d'évaluer la symétrie de la marche ou de la course ;
- le nombre de cycles nécessaires à une séquence ou une sous séquence de marche ou de course.

**13.** Procédé selon la revendication précédente, **caractérisé en ce qu'**il est calculé pour déterminer la régularité de la marche ou course:

- la moyenne de toutes les cellules (i, j) de la matrice carrée M(i,j) en excluant les cellules i=j,
- la déviation standard de toutes les cellules (i, j) de la matrice carrée M(i,j) en excluant les cellules i=j,
- la moyenne des cellules i allant de 1 à Nd (ou de 1 à Ng), et j allant de Nd+1 à Nd+Ng (j allant de Ng+1 à Nd+Ng).

**14.** Procédé selon l'une des revendications précédentes, **caractérisé en ce que** la grandeur physique mesurée est choisie parmi la liste suivante : la norme de l'accélération, la norme de l'accélération sans la composante de gravité terrestre, la vitesse, la vitesse angulaire, le déplacement, la position ou une force exercée par la personne en déplacement sur un dispositif.

**15.** Procédé selon l'une des revendications précédentes 1 à 14, dans lequel les signaux temporels bruts sont traités et séparés préalablement au calcul du coefficient de similarité automatiquement ou manuellement à l'étape de calcul (iii).

**16.** Dispositif (1) permettant par une représentation visuelle :

- l'analyse temporelle de la régularité et de la symétrie d'une séquence de N cycles de marche ou de course d'une même personne, chacun desdits cycles étant séparé en deux phases dont une phase d'appui où le pied de ladite personne est en contact avec le sol et une phase oscillante ou pendulaire où le talon de la personne est en l'air, en comparant chaque cycle pris individuellement par rapport à chacun des autres cycles pris individuellement,
- et la détermination de la présence et du nombre de cycles erratiques ainsi que le nombre de cycles nécessaires à la réalisation des régimes de la séquence de marche ou de course, et à quel moment dans la séquence ces cycles erratiques et régimes sont atteints,

le dispositif (1) comportant :

• des capteurs de mesure (2) de signaux temporels bruts inertiels ou relatifs à une grandeur physique de déplacement d'au moins un segment anatomique de la personne,
• une unité de traitement et de calcul (3), reliée aux capteurs de mesure (2), et agencée pour :

- traiter et séparer les signaux temporels bruts en signaux temporels distincts Ci, chaque signal temporel Ci étant une série temporelle de points de la grandeur physique mesurée et présentant une forme, une amplitude et une durée donnée, la série Ci étant associée à un cycle de marche ou de course donné i de la personne, la séparation des signaux temporels bruts en signaux temporels distincts Ci étant mise en œuvre au moyen d'un repérage, dans les signaux temporels bruts, des instants où le talon de la personne touche le sol;
- calculer au moins un coefficient de similarité entre le signal Ci associé au cycle de marche ou de course i, avec un autre signal Cj associé à un cycle de marche ou de course j de la même personne, le coefficient de similarité étant :

un coefficient de similarité de la forme fij des deux signaux Ci et Cj qui est indépendant de l'amplitude et de la durée, avec fij=fji, ou
un coefficient de similarité de l'amplitude aij des deux signaux Ci et Cj qui est indépendant de la forme et de la durée, avec aij=aji, ou
un coefficient de similarité de la durée dij des deux signaux Ci et Cj qui est indépendant de l'amplitude et de la forme, avec dij=dji,

- ranger dans une matrice carrée M(i,j), la valeur du coefficient de similarité fij, aij ou dij à la ligne i et à la colonne j;

avec i et j entiers naturels variant de 1 à N, les N cycles de marche ou de course étant ordonnés chronologiquement, suivant leur ordre dans la séquence de marche ou de course,

• des moyens d'affichage (4) reliés à l'unité de traitement et de calcul (3), et affichant la matrice carrée M(i,j) avec les valeurs du coefficient de similarité représentées dans les cellules (i,j) de la matrice carrée M(i,j), par une représentation visuelle de la valeur du coefficient de similarité fij, aij ou dij située dans un intervalle entre deux valeurs extrêmes, de façon continue sans seuils,

pour permettre de visualiser simultanément :

- la similarité entre l'ensemble des cycles de marche ou de course i et j de la même personne, et
- la présence et le nombre de cycles erratiques et le nombre de cycles nécessaires à la réalisation des régimes de la séquence de marche ou de course, et à quel moment dans la séquence ces cycles erratiques et régimes sont atteints.

**17.** Dispositif selon la revendication précédente 16, **caractérisé en ce que** les moyens de traitement et de calcul déterminent le coefficient de similarité entre le signal Ci relatif à la marche ou à la course du pied droit (respectivement du pied gauche) de la personne, et le signal Cj relatif à la marche ou à la course du pied droit (respectivement du pied gauche) de la même personne, et **en ce que** N=Nd (respectivement Ng) qui est le nombre de cycles du pied droit (respectivement le nombre de cycles du pied gauche).

**18.** Dispositif selon la revendication précédente 17, **caractérisé en ce que** les moyens de traitement et de calcul déterminent également le coefficient de similarité entre le signal Ci relatif à la marche ou à la course du pied droit de la personne, et le signal Cj relatif à la marche ou à la course du pied gauche de la même personne, et **en ce que** le nombre de cycles N est égal à la somme du nombre de cycles du pied droit et du nombre de cycles du pied gauche : N=Nd +Ng.

**19.** Dispositif selon l'une des revendications précédentes 15 à 18, **caractérisé en ce que** les régimes de marche ou de course déterminés sont : un ou plusieurs régimes établis, initiation de la séquence de marche ou de course, ou demi-tour.

**20.** Dispositif selon l'une des revendications précédentes 15 à 19, **caractérisé en ce que** dans l'étape d'affichage (iv), il est représenté chaque valeur du coefficient de similarité par une couleur située dans une échelle continue graduée en correspondance avec l'échelle des coefficients de similarité.

**21.** Dispositif selon l'une des revendications précédentes 15 à 20, **caractérisé en ce que** les capteurs de mesure (2) sont : accéléromètre, gyroscope, électromyographie, semelles de pression, dispositif d'acquisition cinématique infrarouge ou plate-forme de force, et **en ce que** la grandeur physique mesurée est choisie parmi la liste suivante : la norme de l'accélération, la norme de l'accélération sans la composante de gravité terrestre, la vitesse, la vitesse angulaire, le déplacement, la position ou une force exercée par la personne en déplacement sur un dispositif.

**Patentansprüche**

**1.** Verfahren, das durch eine visuelle Darstellung Folgendes ermöglicht:

• das zeitliche Analysieren der Gleichmäßigkeit und der Symmetrie einer Sequenz von N Gang- oder Laufzyklen derselben Person, wobei jeder dieser Zyklen in zwei Phasen unterteilt ist, von denen eine eine Stützphase bildet, in der der Fuß der Person den Boden berührt, und die andere eine Schwing- oder Pendelphase ist, in der die Ferse der Person in der Luft ist, indem jeder einzeln genommene Zyklus mit jedem der anderen einzeln genommenen Zyklen verglichen wird,
• und Bestimmen des Vorhandenseins und der Anzahl der unregelmäßigen Zyklen sowie der Anzahl der Zyklen, die zum Erreichen der Gang- oder Laufregime erforderlich sind, und zu welchem Zeitpunkt in der Sequenz diese unregelmäßigen Zyklen und Gang- oder Laufregime erreicht werden,
umfassend die folgenden Schritte:

• einen Schritt (i) des Messens von rohen inertiellen Zeitsignalen oder Zeitsignalen, die sich auf eine physikalische Verschiebungsgröße von mindestens einem anatomischen Segment der Person beziehen,
• einen Verarbeitungs- und Berechnungsschritt, unterteilt in:

- einen Schritt (ii) des Verarbeitens und Separierens der rohen Zeitsignale in separate Zeitsignale Ci, um das Gehen oder Laufen in Gang- oder Laufzyklen aufzuteilen, wobei jedes separate Zeitsignal Ci eine Zeitreihe von Punkten der gemessenen physikalischen Größe bildet und eine bestimmte Form, Amplitude und Dauer aufweist, wobei die Reihe Ci einem bestimmten Gang- oder Laufzyklus i der Person zugeordnet ist, und wobei die Trennung der rohen Zeitsignale in separate Zeitsignale Ci durch Kennzeichnung der Zeitpunkte in den rohen Zeitsignalen erfolgt, an denen die Ferse der Person den Boden berührt;
- einen Schritt (iii) des Berechnens mindestens eines Ähnlichkeitskoeffizienten zwischen dem Signal Ci, das dem Gang- oder Laufzyklus i zugeordnet ist, und einem anderen Signal Cj, das dem Gang- oder Laufzyklus j derselben Person zugeordnet ist, wobei der Ähnlichkeitskoeffizient jeweils:

• ein Ähnlichkeitskoeffizient der Form fij der beiden Signale Ci und Cj ist, der unabhängig von Amplitude und Dauer ist, mit fij=fji, oder

• ein Ähnlichkeitskoeffizient der Amplitude aij der beiden Signale Ci und Cj ist, der unabhängig von Form und Dauer ist, mit aij=aji, oder
• ein Ähnlichkeitskoeffizient der Dauer dij der beiden Signale Ci und Cj ist, der unabhängig von Form und Dauer ist, mit dij=dji;
• einen Schritt (iv) des Speicherns des Werts des Ähnlichkeitskoeffizienten fij, aij oder dij in der Zeile i und der Spalte j einer quadratischen Matrix M(i,j), wobei i und j natürliche Ganzzahlen von 1 bis N sind und die N Gang- oder Laufzyklen chronologisch der Reihenfolge in der Gang- oder Laufsequenz folgen;
• einen Schritt (v) des Anzeigens der quadratischen Matrix M(i,j) mit den Werten des Ähnlichkeitskoeffizienten fij, aij oder dij in den Zellen (i,j), durch eine visuelle Darstellung des Werts des Ähnlichkeitskoeffizienten fij, aij oder dij, der sich kontinuierlich in einem Intervall zwischen zwei Extremwerten befindet, ohne Schwellenwerte, um simultan zu ermöglichen:

- die Ähnlichkeit zwischen allen Gang- oder Laufzyklen i und j derselben Person,
- die Anzahl der unregelmäßigen Zyklen und der Anzahl der Zyklen, die zum Erreichen der Regime der Gang- oder Laufsequenz erforderlich sind, und zu welchem Zeitpunkt in der Sequenz diese unregelmäßigen Zyklen und Regime erreicht werden.

**2.** Verfahren nach dem vorhergehenden Anspruch 1, **dadurch gekennzeichnet, dass** im Verarbeitungs- und Berechnungsschritt der Ähnlichkeitskoeffizient zwischen dem Signal Ci bezogen auf das Gehen oder Laufen des rechten Fußes (beziehungsweise des linken Fußes) der Person und dem Signal Cj bezogen auf das Gehen oder Laufen des rechten Fußes (beziehungsweise des linken Fußes) derselben Person bestimmt wird, und dass N = Nd (beziehungsweise Ng) die Anzahl der Zyklen des rechten Fußes (beziehungsweise des linken Fußes) ist.

**3.** Verfahren nach dem vorhergehenden Anspruch 2, **dadurch gekennzeichnet, dass** im Verarbeitungs- und Berechnungsschritt auch der Ähnlichkeitskoeffizient zwischen dem Signal Ci bezogen auf das Gehen oder Laufen des rechten Fußes der Person und dem Signal Cj bezogen auf das Gehen oder Laufen des linken Fußes derselben Person bestimmt wird, und dass N = Nd + Ng ist.

**4.** Verfahren nach einem der vorhergehenden Ansprüche, **dadurch gekennzeichnet, dass** die bestimmten Gang- oder Laufregime Folgendes sind: ein oder mehrere etablierte Regime, Beginn der Gang- oder Laufsequenz, oder eine halbe Umdrehung.

**5.** Verfahren nach einem der vorhergehenden Ansprüche, **dadurch gekennzeichnet, dass** im Anzeigeschritt (v) jeder Wert des Ähnlichkeitskoeffizienten durch eine Farbe dargestellt wird, die in einer kontinuierlich abgestuften Skala entsprechend der Skala der Ähnlichkeitskoeffizienten positioniert ist.

**6.** Verfahren nach dem vorhergehenden Anspruch 5, **dadurch gekennzeichnet, dass**:

• im Verarbeitungs- und Berechnungsschritt die drei unterschiedlichen Ähnlichkeitskoeffizienten fij, aij, dij berechnet werden, wobei jeder Ähnlichkeitskoeffizient fij, aij, dij der gleichen Farbskala wie die anderen Ähnlichkeitskoeffizienten zugeordnet ist, wobei jede Farbe unabhängig vom verwendeten Ähnlichkeitskoeffizienten die gleiche Bedeutung hat;
• im Anzeigeschritt (v) die Werte der Ähnlichkeitskoeffizienten fij, aij, dij in verschiedenen quadratischen Matrizen (F(i,j), A(i,j), D(i,j)) dargestellt werden;

wobei die Ähnlichkeitskoeffizienten so gewählt werden,

• dass nach der Berechnung alle Werte der Ähnlichkeitskoeffizienten fij, aij, dij im gleichen Intervall [a; b] liegen, und
• dass die Signale Ci und Cj umso ähnlicher sind, je höher die Werte der Ähnlichkeitskoeffizienten fij, aij, dij sind.

**7.** Verfahren nach einem der vorhergehenden Ansprüche, **dadurch gekennzeichnet, dass** der Ähnlichkeitskoeffizient ein Formkoeffizient fij ist, und dass im Verarbeitungs- und Berechnungsschritt:

• jedes Zeitsignal Ci und jedes Zeitsignal Cj in ihrer Dauer normalisiert wird, sodass beide Signale die gleiche Dauer aufweisen;
• jedes Zeitsignal Ci in seiner Amplitude normalisiert wird;

- ein Ähnlichkeitskoeffizient der Form fij zwischen jedem normalisierten Signal C'i und einem anderen normalisierten Signal C'j für alle Gang- oder Laufzyklen berechnet wird.

8. Verfahren nach dem vorhergehenden Anspruch, **dadurch gekennzeichnet, dass** der Ähnlichkeitskoeffizient fij der Form der Pearson-Koeffizient ist, wobei die Werte des Ähnlichkeitskoeffizienten auf 0 zurückgesetzt werden, wenn der Wert des Pearson-Koeffizienten kleiner als null ist.

9. Verfahren nach einem der vorhergehenden Ansprüche, **dadurch gekennzeichnet, dass**

   - der Ähnlichkeitskoeffizient ein Ähnlichkeitskoeffizient der Dauer dij ist,
   - die Verarbeitungs- und Recheneinheit (3) für Folgendes angeordnet ist:

     - Berechnung der Dauer Di des Signals Ci und der Dauer Dj des Signals Cj;
     - Berechnung des Ähnlichkeitskoeffizienten der Dauer dij als Verhältnis der kleinsten der beiden Dauern Di, Dj zur größten Dauer.

10. Verfahren nach einem der vorhergehenden Ansprüche, **dadurch gekennzeichnet, dass**

    - der Ähnlichkeitskoeffizient ein Ähnlichkeitskoeffizient der Amplitude aij ist,
    - die Verarbeitungs- und Recheneinheit (3) für Folgendes angeordnet ist:

      - Berechnung der Amplitude Ai des Signals Ci und der Amplitude Aj des Signals Cj;
      - Berechnung des Ähnlichkeitskoeffizienten der Amplitude aij als Verhältnis der kleinsten zur größten Amplitude.

11. Verfahren nach dem vorhergehenden Anspruch, **dadurch gekennzeichnet, dass** die Amplitude Ai, Aj die Standardabweichung oder der Effektivwert ist.

12. Verfahren nach einem der vorhergehenden Ansprüche, **dadurch gekennzeichnet, dass** Folgendes berechnet wird:

    - Parameter, die es ermöglichen, die Gleichmäßigkeit des Gehens oder des Laufens zu beurteilen;
    - Parameter, die es ermöglichen, die Symmetrie des Gehens oder des Laufens zu beurteilen;
    - die Anzahl der Zyklen, die für eine Gang- oder Laufsequenz oder Untersequenz erforderlich sind.

13. Verfahren nach einem der vorhergehenden Ansprüche, **dadurch gekennzeichnet, dass** zum Bestimmen der Gleichmäßigkeit des Gehens oder des Laufens Folgendes berechnet wird:

    - der Mittelwert aller Zellen (i, j) der quadratischen Matrix M(i,j) unter Ausschluss der Zellen i = j,
    - die Standardabweichung aller Zellen (i, j) der quadratischen Matrix M(i,j) unter Ausschluss der Zellen i = j,
    - der Mittelwert der Zellen (i,j) mit i von 1 bis Nd (bzw. von 1 bis Ng) und j von Nd+1 bis Nd+Ng (bzw. von Ng+1 bis Nd+Ng).

14. Verfahren nach einem der vorhergehenden Ansprüche, **dadurch gekennzeichnet, dass** die gemessene physikalische Größe aus der folgenden Liste ausgewählt wird: Beschleunigungsnorm, Beschleunigungsnorm ohne die Komponente der Erdanziehungskraft, Geschwindigkeit, Winkelgeschwindigkeit, Verschiebung, Position, Kraft, die von der sich bewegenden Person auf eine Vorrichtung ausgeübt wird.

15. Verfahren nach einem der vorhergehenden Ansprüche 1 bis 14, wobei die rohen Zeitsignale vor der Berechnung des Ähnlichkeitskoeffizienten automatisch oder manuell im Berechnungsschritt (iii) verarbeitet und getrennt werden.

16. Vorrichtung (1), die durch eine visuelle Darstellung Folgendes ermöglicht:

    - das zeitliche Analysieren der Gleichmäßigkeit und der Symmetrie einer Sequenz von N Gang- oder Laufzyklen derselben Person, wobei jeder dieser Zyklen in zwei Phasen unterteilt ist, von denen eine eine Stützphase bildet, in der der Fuß der Person den Boden berührt, und die andere eine Schwing- oder Pendelphase ist, in der die Ferse der Person in der Luft ist, indem jeder einzeln genommene Zyklus mit jedem der anderen einzeln genommenen Zyklen verglichen wird,
    - und Bestimmen des Vorhandenseins und der Anzahl der unregelmäßigen Zyklen sowie der Anzahl der Zyklen, die zum Erreichen der Gang- oder Laufregime erforderlich sind, und zu welchem Zeitpunkt in der Sequenz diese unregelmäßigen Zyklen und Gang- oder Laufregime erreicht werden,

wobei die Vorrichtung (1) Folgendes umfasst:

• Sensoren (2) zum Messen von rohen inertiellen Zeitsignalen oder Zeitsignalen, die sich auf eine physikalische Verschiebungsgröße von mindestens einem anatomischen Segment der Person beziehen,
• eine Verarbeitungs- und Recheneinheit (3), die mit den Messsensoren (2) verbunden und für Folgendes angeordnet ist:

- Verarbeiten und Trennen der rohen Zeitsignale in separate Zeitsignale Ci, wobei jedes separate Zeitsignal Ci eine Zeitreihe von Punkten der gemessenen physikalischen Größe bildet und eine bestimmte Form, Amplitude und Dauer aufweist, wobei die Reihe Ci einem bestimmten Gang- oder Laufzyklus i der Person zugeordnet ist, und wobei die Trennung der rohen Zeitsignale in separate Zeitsignale Ci durch Kennzeichnung der Zeitpunkte in den rohen Zeitsignalen erfolgt, an denen die Ferse der Person den Boden berührt;
- Berechnung mindestens eines Ähnlichkeitskoeffizienten zwischen dem Signal Ci, das dem Gang- oder Laufzyklus i zugeordnet ist, und einem anderen Signal Cj, das einem Gang- oder Laufzyklus j derselben Person zugeordnet ist, wobei der Ähnlichkeitskoeffizient jeweils:

• ein Ähnlichkeitskoeffizient der Form fij der beiden Signale Ci und Cj ist, der unabhängig von Amplitude und Dauer ist, mit fij = fji, oder
• ein Ähnlichkeitskoeffizient der Amplitude aij der beiden Signale Ci und Cj ist, der unabhängig von Form und Dauer ist, mit aij = aji, oder
• ein Ähnlichkeitskoeffizient der Dauer dij der beiden Signale Ci und Cj ist, der unabhängig von Amplitude und Form ist, mit dij = dji;

- Speichern des Werts des Ähnlichkeitskoeffizienten fij, aij oder dij in Zeile i und Spalte j einer quadratischen Matrix M(i,j), wobei i und j natürliche Ganzzahlen von 1 bis N sind und die N Gang- oder Laufzyklen chronologisch nach ihrer Reihenfolge in der Gang- oder Laufsequenz geordnet sind;

• Anzeigemittel (4), die mit der Verarbeitungs- und Recheneinheit (3) verbunden sind, zum Anzeigen der quadratischen Matrix M(i,j) mit den in den Zellen (i,j) dargestellten Werten des Ähnlichkeitskoeffizienten durch eine visuelle Darstellung des Werts des Ähnlichkeitskoeffizienten fij, aij oder dij, der sich kontinuierlich in einem Intervall zwischen zwei Extremwerten befindet, ohne Schwellenwerte, um simultan Folgendes zu ermöglichen:

- die Ähnlichkeit zwischen allen Gang- oder Laufzyklen i und j derselben Person,
- das Vorhandensein und die Anzahl der unregelmäßigen Zyklen sowie die Anzahl der Zyklen, die zum Erreichen der Gang- oder Laufregime erforderlich sind, und zu welchem Zeitpunkt in der Sequenz diese unregelmäßigen Zyklen und Regime erreicht werden.

**17.** Vorrichtung nach dem vorhergehenden Anspruch 16, **dadurch gekennzeichnet, dass** die Verarbeitungs- und Recheneinheit (3) den Ähnlichkeitskoeffizienten zwischen dem Signal Ci, bezogen auf das Gehen oder Laufen des rechten Fußes ( bzw. des linken Fußes) der Person, und dem Signal Cj, bezogen auf das Gehen oder Laufen desselben Fußes derselben Person, bestimmt, und dass N = Nd ( bzw. Ng) die Anzahl der Zyklen des rechten Fußes ( bzw. des linken Fußes) ist.

**18.** Vorrichtung nach dem vorhergehenden Anspruch 17, **dadurch gekennzeichnet, dass** die Verarbeitungs- und Recheneinheit (3) auch den Ähnlichkeitskoeffizienten zwischen dem Signal Ci, bezogen auf das Gehen oder Laufen des rechten Fußes der Person, und dem Signal Cj, bezogen auf das Gehen oder Laufen des linken Fußes derselben Person, bestimmt, und dass N = Nd + Ng ist.

**19.** Vorrichtung nach einem der vorhergehenden Ansprüche 15 bis 18, **dadurch gekennzeichnet, dass** die bestimmten Gang- oder Laufregime Folgendes sind: ein oder mehrere etablierte Regime, Beginn der Gang- oder Laufsequenz oder eine halbe Umdrehung.

**20.** Vorrichtung nach einem der vorhergehenden Ansprüche 15 bis 19, **dadurch gekennzeichnet, dass** im Anzeigeschritt (iv) jeder Wert des Ähnlichkeitskoeffizienten durch eine Farbe dargestellt wird, die in einer kontinuierlich abgestuften Skala entsprechend der Skala der Ähnlichkeitskoeffizienten positioniert ist.

**21.** Vorrichtung nach einem der vorhergehenden Ansprüche 15 bis 20, **dadurch gekennzeichnet, dass** die Messsensoren (2) Beschleunigungsmesser, Gyroskop, Elektromyographie, Drucksohlen, Infrarot-kinematische Erfassungsvorrichtung oder Kraftplattform sind, und dass die gemessene physikalische Größe aus der folgenden Liste ausgewählt wird: Beschleunigungsnorm, Beschleunigungsnorm ohne die Komponente der Erdanziehungskraft, Geschwindigkeit, Winkelgeschwindigkeit, Verschiebung, Position, Kraft, die von der sich bewegenden Person auf eine Vorrichtung ausgeübt wird.

## Claims

**1.** A method allowing, by a visual representation:

- the temporal analysis of the regularity and symmetry of a sequence of N cycles of walking or running of the same person, each of said cycles being separated into two phases of which one is a support phase where the foot of said person is in contact with the ground and the other is an oscillating or pendulum phase where the heel of the person is in the air, by comparing each cycle considered individually with each of the other cycles considered individually,
- and determining the presence and the number of erratic cycles as well as the number of cycles required to achieve the walking or running regimes, and at what moment in the sequence these erratic cycles and walking regimes are reached,

including the following steps:

- a step (i) of measuring raw inertial time signals or time signals relating to a physical displacement variable of at least one anatomical segment of the person,
- a processing and calculation step, subdivided into:
    - a step (ii) of processing and separating the raw time signals into distinct time signals Ci in order to break down the walking or running into walking or running cycles, each distinct time signal Ci being a time series of points of the measured physical variable and having a given shape, amplitude and duration, the series Ci being associated with a given walking or running cycle i of the person, the separation of the raw time signals into distinct time signals Ci being implemented by means of a marking, in the raw time signals, of the time points when the heel of the person touches the ground;
    - a step (iii) of calculating at least one coefficient of similarity between the signal Ci associated with the walking or running cycle i, with another signal Cj associated with a walking or running cycle j of the same person, the coefficient of similarity being:

        a coefficient of similarity of the form fij of the two signals Ci and Cj which is independent of the amplitude and the duration, with fij=fji, or
        a coefficient of similarity of the amplitude aij of the two signals Ci and Cj which is independent of the form and the duration, with aij=aji, or
        a coefficient of similarity of the duration dij of the two signals Ci and Cj which is independent of the form and the duration, with dij=dji,

a step (iv) of storing into a square matrix M(i,j), the value of the coefficient of similarity fij, aij or dij in the row i and in the column j; with i and j natural integers varying from 1 to N, the N walking or running cycles being chronologically ordered according to their order in the walking or running sequence,

- a step (v) of displaying the square matrix M(i,j) with the values of the coefficient of similarity fij, aij or dij represented in the cells (i,j) of the square matrix M(i,j), by visual representation of the value of the coefficient of similarity fij, aij or dij located in an interval between two extreme values, continuously with no thresholds, to allow the visual and simultaneous determination of:
    - the similarity between all of the walking or running cycles i and j of the same person, by comparing each cycle considered individually with each of the other cycles considered individually,
    - the number of erratic cycles and the number of cycles required to achieve the regimes of the walking or running sequence, and at what moment in the sequence these erratic cycles and regimes are reached.

2. The method according to the preceding claim, **characterized in that**, in the processing and calculation step, the coefficient of similarity is determined between the signal Ci relating to the walking or running of the right foot (respectively of the left foot) of the person, and the signal Cj relating to the walking or running of the right foot (respectively of the left foot) of the same person, and **in that** N=Nd (respectively Ng) which is the number of cycles of the right foot (respectively the number of cycles of the left foot).

3. The method according to the preceding claim 2, **characterized in that**, in the processing and calculation step, the coefficient of similarity between the signal Ci relating to the walking or running of the right foot of the person, and the signal Cj relating to the walking or running of the left foot of the same person, is also determined, and **in that** the number of cycles N is equal to the sum of the number of cycles of the right foot and the number of cycles of the left foot: N=Nd+Ng.

4. The method according to one of the preceding claims, **characterized in that** the determined walking or running regimes are: one or more established regime(s), initiation of the walking or running sequence, or a half-turn.

5. The method according to one of the preceding claims, **characterized in that**, in the display step (v), each value of the coefficient of similarity is represented by a color positioned in a continuous graduated scale corresponding to the scale of the coefficients of similarity.

6. The method according to the preceding claim 5, **characterized in that**:

   - in the processing and calculation step, the three different coefficients of similarity fij, aij, dij are calculated, each coefficient of similarity fij, aij, dij being associated with the same color scale as that of the other coefficients of similarity, each color having the same meaning of similarity regardless of the coefficient of similarity used;
   - in the display step (v), the values of the coefficients of similarity fij, aij, dij are presented in different square matrices (F(i,j), A(i,j), D(i,j));

   the coefficients of similarity being selected:

   - so that, once calculated, all of the values of the coefficients of similarity fij, aij, dij are in the same interval [a; b] and
   - so that the higher the values of the coefficients fij, aij, dij, the more similar the signals Ci and Cj will be.

7. The method according to one of the preceding claims, **characterized in that** the coefficient of similarity is a form coefficient fij, and **in that** it is, in the processing and calculation step:

   - normalize in duration each time signal Ci with each time signal Cj so that the two signals Ci and Cj have the same duration;
   - normalize in amplitude each time signal Ci;
   - calculate a coefficient of similarity of the form fij between each normalized signal C'i, with another normalized signal C'j, and that being so for all of the walking or running cycles.

8. The method according to the preceding claim, **characterized in that** the coefficient of similarity of the form fij is the Pearson coefficient, the values of the coefficient of similarity being reduced to 0 in the case of a Pearson coefficient value lower than zero.

9. The method according to one of the preceding claims, **characterized in that**

   - the coefficient of similarity is a coefficient of similarity of duration dij,
   - the processing and calculation unit (3) is arranged for

     » calculating a duration Di, of the signal Ci and of the duration Dj of the signal Cj,
     • calculating the coefficient of similarity of duration dij which is the ratio of the shortest of the two durations Di, Dj divided by the longest duration Di, Dj.

10. The method according to one of the preceding claims, **characterized in that**

   - the coefficient of similarity is a coefficient of similarity of amplitude aij,
   - and, the processing and calculation unit (3) is arranged for

• calculating an amplitude Ai, Aj of the signal Ci and of the signal Cj,
• calculating the coefficient of similarity of amplitude aij which is the ratio of the smallest amplitude Ai, Aj divided by the largest amplitude Ai, Aj.

**11.** The method according to the preceding claim, **characterized in that** the amplitude Ai, Aj is the standard deviation or the effective value.

**12.** The method according to one of the preceding claims, **characterized in that** the following is calculated:

- parameters allowing assessing the regularity of the walking or running;
- parameters allowing assessing the symmetry of the walking or running;
- the number of cycles required for a walking or running sequence or sub-sequence.

**13.** The method according to the preceding claim, **characterized in that**, to determine the regularity of the walking or running, the following is calculated:

- the average of all cells (i, j) of the square matrix M(i,j) excluding the cells i=j,
- the standard deviation of all cells (i, j) of the square matrix M(i,j) excluding the cells i=j,

the average of the cells i ranging from 1 to Nd (or from 1 to Ng), and j ranging from Nd+1 to Nd+Ng (or j ranging from Ng+1 to Nd+Ng).

**14.** The method according to one of the preceding claims, **characterized in that** the measured physical variable is selected from among the following list: the acceleration norm, the acceleration norm without the terrestrial gravity component, the speed, the angular speed, the displacement, the position or a force exerted by the person in motion on a device.

**15.** The method according to one of the preceding claims 1 to 14, wherein the raw time signals are processed and separated automatically or manually prior to the calculation of the coefficient of similarity automatically or manually in the calculation step (iii).

**16.** A device (1) allowing, by a visual representation:

• the temporal analysis of the regularity and symmetry of a sequence of N walking or running cycles of the same person, each of said cycles being separated into two phases of which one is a support phase where the foot of said person is in contact with the ground and the other is an oscillating or pendulum phase where the heel of the person is in the air, by comparing each cycle considered individually with each of the other cycles considered individually,
• and determining the presence and the number of erratic cycles as well as the number of cycles required to achieve the regimes of the walking or running sequence, and at what moment in the sequence these erratic cycles and regimes are reached,

the device (1) comprising:

• sensors (2) for measuring raw inertial time signals or time signals relating to a physical displacement variable of at least one anatomical segment of the person,
• a processing and computing unit (3), connected to the measurement sensors (2), arranged for:

◦ processing and separating the raw time signals into distinct time signals Ci, each time signal Ci being a time series of points of the measured physical variable and having a given form, amplitude, and duration, the series Ci being associated with a given walking or running cycle i of the person, the separation of the raw time signals into distinct time signals Ci being implemented by marking, in the raw time signals, the time points when the heel of the person touches the ground;
◦ calculating at least one coefficient of similarity between the signal Ci associated with the walking or running cycle i, with another signal Cj associated with a walking or running cycle j of the same person, the coefficient of similarity being:

▪ a coefficient of similarity of the form fij of the two signals Ci and Cj which is independent of the amplitude and the duration, with fij=fji, or

- ▪ a coefficient of similarity of the amplitude aij of the two signals Ci and Cj which is independent of the form and the duration, with aij=aji, or
- ▪ a coefficient of similarity of the duration dij of the two signals Ci and Cj which is independent of the amplitude and the form, with dij=dji;

◦ storing in a square matrix M(i,j), the value of the coefficient of similarity fij, aij or dij in the row i and in the column j, with i and j natural integers varying from 1 to N, the N walking or running cycles being chronologically ordered according to their order in the walking or running sequence;

• display means (4) connected to the processing and calculation unit (3), and displaying the square matrix M(i,j) with the values of the coefficient of similarity represented in the cells (i,j) of the square matrix M(i,j), by visual representation of the value of the coefficient of similarity fij, aij or dij located in an interval between two extreme values, continuously with no thresholds, to allow simultaneous visualization of:

◦ the similarity between all of the walking or running cycles i and j of the same person,
◦ the presence and the number of erratic cycles and the number of cycles required to achieve the regimes of the walking or running sequence, and at what moment in the sequence these erratic cycles and regimes are reached.

**17.** The device according to the preceding claim 16, **characterized in that** the processing and calculation means determine the coefficient of similarity between the signal Ci relating to the walking or running of the right foot (respectively of the left foot) of the person, and the signal Cj relating to the walking or running of the right foot (respectively of the left foot) of the same person, and **in that** N=Nd (respectively Ng) which is the number of cycles of the right foot (respectively the number of cycles of the left foot).

**18.** The device according to the preceding claim 17, **characterized in that** the processing and calculation means also determine the coefficient of similarity between the signal Ci relating to the walking or running of the right foot of the person, and the signal Cj relating to the walking or running of the left foot of the same person, and **in that** the number of cycles N is equal to the sum of the number of cycles of the right foot and the number of cycles of the left foot: N=Nd+Ng.

**19.** The device according to one of the preceding claims 15 to 18, **characterized in that** the determined walking or running regimes are: one or more established regime(s), initiation of the walking or running sequence, or a half-turn.

**20.** The device according to one of the preceding claims 15 to 19, **characterized in that**, in the display step (iv), each value of the coefficient of similarity is represented by a color positioned in a continuous graduated scale corresponding to the scale of the coefficients of similarity.

**21.** The device according to one of the preceding claims 15 to 20, **characterized in that** the measurement sensors (2) are: accelerometer, gyroscope, electromyography, pressure insoles, infrared kinematic acquisition device or force platform, and **in that** the measured physical variable is selected from among the following list: the acceleration norm, the acceleration norm without the terrestrial gravity component, the velocity, the angular velocity, the displacement, the position or a force exerted by the person in motion on a device.

1
2
3
4
I
II
III
IV
V
VI
3
1
1 amplitude
2 durée
3 forme
2

FIG. 1A

FIG. 1B

FIG. 2

M (i,j)
N
cycle i
1
0,5
0
s
g

FIG. 3

Forme
$P(\tilde{C}_i, \tilde{C}_j) = \frac{cov(\tilde{C}_i, \tilde{C}_j)}{std(\tilde{C}_i)std(\tilde{C}_j)}$
F (i,j)
Sujet sain
Sujet parkinsonien
Cycles
Pied gauche
Pied droit
0


FIG. 4A

FIG. 4B

Amplitude
$A(C_i , C_j)=\min(\frac{std(C_i)}{std(C_j)}, \frac{std(C_j)}{std(C_i)})$
A (i,j)
Sujet sain
A (i,j)
Sujet parkinsonien
Cycles
Pied gauche
Pied droit
0
Pied droit
Pied gauche
Pied droit
Pied gauche


FIG. 5A

FIG. 5B

Durée
$D(C_i, C_j) = \min\left(\frac{|C_i|}{|C_j|}, \frac{|C_j|}{|C_i|}\right)$
D (i,j)
Sujet sain
Sujet parkinsonien
Pied gauche
Pied droit
Cycles
0

FIG. 6A

FIG. 6B

Exemple pour le pied droit
Signaux bruts
$acc_{droit}$
g
1
0
0
5
10
15
20
s
x
y
z
Signaux bruts sans gravité
$accfree_{droit} = \begin{pmatrix} acc_{droit,x} \\ acc_{droit,y} \\ acc_{droit,z} \end{pmatrix} - \begin{pmatrix} accimmobile_{droit,x} \\ accimmobile_{droit,y} \\ accimmobile_{droit,z} \end{pmatrix}$
$accfree_{droit}$
g
1
0
0
5
10
15
20
s

FIG. 7

FIG. 8

Norme de l'accélération
$S_{droit} = \sqrt{accfree_{droit,x}^{2} + aacfree_{droit,y}^{2} + accfree_{droit,z}^{2}}$
$S_{droit}$
g
1
0
0
5
10
15
20
s

FIG. 9

$S_{droit}$
Pied droit
$\tau_{droit,1}$
$\tau_{droit,2}$
$\tau_{droit,N_d}$
$\tau_{droit,N_d+1}$
1
g
0
0
10
20
s
FIG. 10A
$S_{gauche}$
Pied gauche
$\tau_{gauche,1}$
$\tau_{gauche,2}$
$\tau_{gauche,N_g}$
$\tau_{gauche,N_g+1}$
0
10
20
s
FIG. 10B
Détection de pas
Concaténation
$C_1$
$C_2$
$C_{N_d}$
$C_{N_d+1}$
$C_{N_d+2}$
$C_{N_d+N_g}$
∀i
FIG. 10C
$C_i$
1
g
0
0 20 40 60 80 100
échantillons
111 échantillons
<<resample>>
$\tilde{C}_i$
1
g
0
0 20 40 60 80 100
échantillons
100 échantillons
FIG. 10D

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- Estimation of gait cycle characteristics by trunk accelerometry. **MOE-NILSSEN R et al.** JOURNAL OF BIOMECHANICS. PERGAMON PRESS, 2004, vol. 37, 121-126
- **CHE-CHANG YANG et al.** Real-Time Gait Cycle Parameter Recognition Using a Wearable Accelerometry System. *SENSORS*, 25 July 2011, vol. 11 (12), 7314-7326
- **YANG MINGJING et al.** iGAIT: An interactive accelerometer based gait analysis system. *COMPUTER METHODS AND PROGRAMS IN BIOMEDICINE*, 2012, vol. 108 (2), ISSN 0169-2607, 715-723